(19) Europäisches Patentamt
European Patent Office
Office européen des brevets

(11) EP 3 655 414 B1

(12) # EUROPEAN PATENT SPECIFICATION

(45) Date of publication and mention of the grant of the patent:
**25.08.2021 Bulletin 2021/34**

(21) Application number: **18742393.4**

(22) Date of filing: **13.07.2018**

(51) Int Cl.:
***C07F 9/54*** *(2006.01)* ***A61K 31/66*** *(2006.01)*
***A61P 35/00*** *(2006.01)*

(86) International application number:
**PCT/CZ2018/050036**

(87) International publication number:
**WO 2019/015701 (24.01.2019 Gazette 2019/04)**

(54) **DEFEROXAMINE DERIVATIVES AS MEDICAMENTS**

DEFEROXAMINDERIVATE ALS MEDIKAMENTE

DERIVES DE LA DEFEXORAMINE EN TANT QUE MEDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2017 EP 17181654**

(43) Date of publication of application:
**27.05.2020 Bulletin 2020/22**

(73) Proprietors:
- **Biotechnologicky Ustav AV CR, V.V.I.**
  **252 50 Vestec (CZ)**
- **Smart Brain S.r.o.**
  **14220 Praha 4 - Krc (CZ)**

(72) Inventors:
- **TRUKSA, Jaroslav**
  **14100 Praha 4 (CZ)**
- **WERNER, Lukas**
  **25169 Brtnice**
  **Velke Popovice (CZ)**
- **STURSA, Jan**
  **14800 Praha 4 (CZ)**

(74) Representative: **Hartvichova, Katerina**
**HARBER IP s.r.o.**
**Dukelskych hrdinu 567/52**
**170 00 Praha 7 (CZ)**

(56) References cited:
**WO-A1-2017/079535**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 655 414 B1

**Description**

Field of Art

The present invention relates to novel deferoxamine derivatives and their use as medicaments, in particular for treatment of cancers.

Background Art

All cells require iron for their DNA synthesis, metabolism, growth and proliferation. Iron represents an indispensable micronutrient required for many enzymatic reactions such as the catalytic activity of ribonucleotide reductase for the synthesis of deoxyribonucleotides. It is also indispensable for mitochondrial respiration, due to its ability to accept and donate electrons and participate in the electron transport chain, thus leading to the generation of the electrochemical gradient across the inner mitochondrial membrane. Iron exists in biological systems mostly as either ferrous or oxidized ferric form. The amount of iron in the cell and organism is tightly balanced, as iron excess is toxic due to generation of highly reactive oxygen species such as hydroxyl radical (*via* the Fenton and Haber-Weiss reactions), thus leading to damage to DNA, lipids and proteins as seen in iron overload disease - hemochromatosis. On the other hand, insufficient amount of iron leads to compromised cellular respiration and systemic anemia.

In biological systems, iron is usually present as a cofactor in the form of heme iron or in the form of Fe-S clusters. Both of them are synthesized in mitochondria, an organelle responsible for oxidative phosphorylation and cellular respiration, and iron-containing proteins are critical components of the electron transport chain. Thus, mitochondria is considered the central player in the cellular iron metabolism and homeostasis.

Since cancer cells show higher demand for iron due to their proliferative nature and altered metabolic needs, an important role of iron in tumour growth and progression is expected. Moreover, high tissue iron has been linked with increased incidence of liver and colorectal cancer. Recently, a breast cancer specific gene signature suggesting an increase in iron uptake and a decrease in iron export has been observed and correlated with poor clinical outcome (Miller,L.D., Coffman,L.G., Chou,J.W., Black,M.A., Bergh,J., D'Agostino,R., Jr., Torti,S.V., & Torti,F.M. (2011) An iron regulatory gene signature predicts outcome in breast cancer. Cancer Research, 71, 6728-6737). In addition, high expression of transferrin receptor 1 has been documented in cells resistant to tamoxifen (Habashy,H.O., Powe,D.G., Staka,C.M., Rakha,E.A., Ball,G., Green,A.R., Aleskandarany,M., Paish,E.C., Douglas,M.R., Nicholson,R.I., Ellis,I.O., & Gee,J.M. (2009) Transferrin receptor (CD71) is a marker of poor prognosis in breast cancer and can predict response to tamoxifen. Breast Cancer Res.Treat., 119, 283-293) and marked alterations in the iron metabolism and a specific iron metabolism-related gene signature in tumour-initiating cells has been recently reported, documenting an important role of iron in various types of cancer (Rychtarcikova,Z., Lettlova,S., Tomkova,V., Korenkova,V., Langerova,L., Simonova,E., Zjablovskaja,P., Alberich-Jorda,M., Neuzil,J., & Truksa,J. (2017) Tumor-initiating cells of breast and prostate origin show alterations in the expression of genes related to iron metabolism. Oncotarget., 8, 6376-6398).

Importantly, iron participates in the regulation of hypoxia inducible factors (HIF), factors often linked to neovascularization and cancer progression, *via* its critical role as a cofactor of prolyl hydroxylases that regulate stability of HIFs (Koh,M.Y., Lemos,R., Jr., Liu,X., & Powis,G. (2011) The hypoxia-associated factor switches cells from HIF-1alpha- to HIF-2alpha-dependent signaling promoting stem cell characteristics, aggressive tumor growth and invasion. Cancer Research, 71, 4015-4027; Peyssonnaux,C., Zinkernagel,A.S., Schuepbach,R.A., Rankin,E., Vaulont,S., Haase,V.H., Nizet,V., & Johnson,R.S. (2007) Regulation of iron homeostasis by the hypoxia-inducible transcription factors (HIFs). Journal of Clinical Investigation, 117, 1926-1932). Similarly, a link between iron and activity of the Wnt/β-catenin signaling pathway has been proposed (Song,S., Christova,T., Perusini,S., Alizadeh,S., Bao,R.Y., Miller,B.W., Hurren,R., Jitkova,Y., Gronda,M., Isaac,M., Joseph,B., Subramaniam,R., Aman,A., Chau,A., Hogge,D.E., Weir,S.J., Kasper,J., Schimmer,A.D., Al-awar,R., Wrana,J.L., & Attisano,L. (2011) Wnt inhibitor screen reveals iron dependence of beta-catenin signaling in cancers. Cancer Research, 71, 7628-7639). Taken together, the current evidence strongly supports the notion that iron plays an important role in carcinogenesis at multiple levels.

Iron chelators have been widely used for treatment of iron overload diseases, with deferoxamine (DFO) being the first prototypical compound (Graziano,J.H. (1978) Iron metabolism and chelation therapy in hemosiderosis. Curr.Top.Hematol., 1, 127-150). More recently, iron chelators, especially DFO, have been shown to induce apoptosis in cancer cells, particularly those of hematopoietic origin, and some studies document curative effect on cancer cells even *in vivo* ( Fukuchi,K., Tomoyasu,S., Tsuruoka,N., & Gomi,K. (1994) Iron deprivation-induced apoptosis in HL-60 cells. FEBS Letters, 350, 139-142; Seligman,P.A., Kovar,J., & Gelfand,E.W. (1992) Lymphocyte proliferation is controlled by both iron availability and regulation of iron uptake pathways. Pathobiology, 60, 19-26; Seligman,P.A., Schleicher,R.B., Siriwardana,G., Domenico,J., & Gelfand,E.W. (1993) Effects of agents that inhibit cellular iron incorporation on bladder cancer cell proliferation. Blood, 82, 1608-1617; White,S., Taetle,R., Seligman,P.A., Rutherford,M., & Trowbridge,I.S. (1990) Combinations of anti-transferrin receptor monoclonal antibodies inhibit human tumor cell growth in vitro and in

vivo: evidence for synergistic antiproliferative effects. Cancer Research, 50, 6295-6301). Yet, the main drawback is a major change in the systemic organismal iron metabolism and consequent death of the experimental animals.

WO 2017/079535 discloses alkylphosphocholine analogs incorporating a chelating moiety chelated to gadolinium. The chelating moieties have varied structures, including a deferoxamine-type structure. The compounds of WO 2017/079535 comprising the gadolinium atom are useful in magnetic resonance imaging and in treating cancer by neutron capture therapy.

The aim of the present invention is to provide compounds selectively affecting the iron metabolism in cancer cells and having curative effects, without showing the undesirable systemic effects.

Disclosure of the Invention

The present invention provides novel substances of general formula I and pharmaceutically acceptable salts thereof,

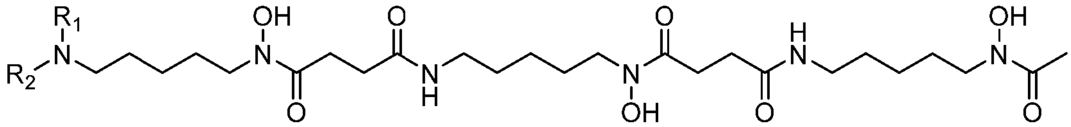
(I)

wherein R1 and R2 is independently selected from the group comprising H; C1-C6 alkyl; C6-C10 aryl; (C1-C6)alkyl(C6-C10)aryl; -C(=O)-R'; -C(=O)OR'; -C(=O)NR'R"; -C(=S)R'; -C(=S)NR'R"; wherein R' and R" are independently selected from the group comprising H, C1-C6 alkoxy, C1-C6 alkyl, C6-C10 aryl, (C1-C6)alkyl(C6-C10)aryl; whereas C1-C6 alkoxy, C1-C6 alkyl, C6-C10 aryl, (C1-C6)alkyl(C6-C10)aryl can be unsubstituted or substituted by one or more substituents selected independently from the group comprising C1-C4 alkyl, N(H or C1-C4 alkyl)$_2$, whereas alkyls are the same or different, phenyl, benzyl, OH, SH, F, Cl, Br, I, C1-C4 alkoxy, C1-C4 acyloxy, C1-C4 mercapto; and substituent of general formula II

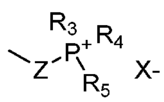
(II)

wherein Z is a linear hydrocarbyl chain selected from alkylene, alkenylene or alkynylene, containing 6 to 20 carbon atoms, preferably 6 to 16 carbon atoms, more preferably 6 to 14 carbon atoms, even more preferably 8 to 12 carbon atoms, most preferably 10 carbon atoms, or preferably 8 to 16 or 10 to 15 carbons, whereas optionally one or more carbon atoms (typically $-CH_2-$ groups) in the hydrocarbyl chain may be replaced by one or more 5-membered or 6-membered aromatic rings or heteroaromatic rings containing the heteroatoms O, S and/or N, preferably phenylenes, triazolylenes or pyridylenes, and/or one or more carbon atoms (typically $-CH_2-$ groups) in the hydrocarbyl chain may be replaced by one or more heteroatoms or heteroatom-containing moieties selected from O, S, NH, N-OH, and whereas the hydrocarbyl chain can be unsubstituted or substituted by one or more substituents selected independently from the group comprising C1-C4 alkyl, N(H or C1-C4 alkyl)$_2$ wherein alkyls are the same or different, phenyl, benzyl, OH, =O, SH, =S, =N-OH, F, Cl, Br, I, C1-C4 alkoxy, C1-C4 acyloxy, C1-C4 mercapto, and

each of R3, R4, R5 is independently selected from the group comprising C1-C10 alkyl, C6-C12 aryl, C6-C12-aryl-C1-C2-alkyl, C5-C12 heteroaryl, C3-C8 cycloalkyl, wherein each of R1, R2, R3 can optionally (and independently from others) be substituted by one or more substituents selected independently from the group comprising C1-C4 alkyl; C1-C4 alkoxy; N(H or C1-C4 alkyl)$_2$, wherein the alkyls are the same or different; OH; =O; SH; =S; =N-OH; F; Cl; Br; I; C1-C4 mercapto,

whereas at least one of R1 and R2 is a substituent of general formula II.

$X^-$ is a pharmaceutically acceptable anion, in particular anion of inorganic or organic acid, particularly suitable are $Cl^-$, $Br^-$, $I^-$, sulphate, phosphate, mesylate, acetate, formiate, succinate, citrate, lactate, tartarate, oxalate, ascorbate, tosylate, but anions of any pharmaceutically acceptable acids can be used.

Pharmaceutically acceptable salts include in particular salts with pharmaceutically acceptable acids, such as 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid

(D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (-L), salicylic acid, sebacic acid, stearic acid, succinic acid, sulfuric acid, tartaric acid (+ L), thiocyanic acid, toluenesulfonic acid (p), undecylenic acid.

Pharmaceutically acceptable esters represent compounds in which the OH groups in the molecule (in particular in the -N(OH)- groups) are esterified by R9-C(=O)- group. R9 can be selected from C1-C20 alkyls, C2-C20 alkenyls.

Preferably, R1 and R2 are independently selected from H, C1-C6 alkyl, and substituent of general formula II.

Preferably, R3, R4, R5 are independently selected from phenyl, benzyl, cyclohexyl, linear C1-C10 alkyl; optionally one or more of R3, R4, R5 may be further substituted by one or two substituents selected independently from the group comprising C1-C4 alkyl; C1-C4 alkoxy; OH; SH; F; Cl; Br; I; C1-C4 mercapto.

Preferably, Z is a linear hydrocarbyl chain selected from alkylene, alkenylene or alkynylene (preferably alkylene), containing 6 to 16 carbon atoms or 6 to 14 carbon atoms, more preferably 8 to 12 carbon atoms or 10 carbon atoms.

Preferably, Z is a linear hydrocarbyl chain selected from alkylene, alkenylene or alkynylene (preferably alkylene), containing 6 to 16 carbon atoms or 6 to 14 carbon atoms, more preferably 8 to 12 carbon atoms or 10 carbon atoms, wherein one or more carbon atoms in the hydrocarbyl chain are replaced by one or more heteroatoms selected from O, S, NH.

Preferably, Z is a linear hydrocarbyl chain selected from alkylene, alkenylene or alkynylene (preferably alkylene), containing 8 to 16 carbon atoms or 10 to 15 carbon atoms, wherein one or more carbon atoms in the hydrocarbyl chain are replaced by one or more heteroatom-containing moieties N-OH and one or more carbon atoms in the hydrocarbyl chain are substituted with =O or =S.

Preferably, Z is a linear hydrocarbyl chain selected from alkylene, alkenylene or alkynylene (preferably alkylene), containing 6 to 16 carbon atoms or 6 to 14 carbon atoms, more preferably 8 to 12 carbon atoms or 10 carbon atoms, wherein one or more carbon atoms in the hydrocarbyl chain are replaced by one or more 5-membered or 6-membered aromatic rings or heteroaromatic rings, preferably phenylenes and/or pyridylenes and/or triazoles.

Preferably, Z is substituted by one or more substituents selected from C1-C4 alkyl; N(H or C1-C4 alkyl)$_2$, wherein the alkyls are the same or different; OH; =O; SH; =S; F; Cl; Br; I; C1-C4 alkoxy; C1-C4 mercapto; more preferably, Z is substituted by one or more substituents selected from OH; =O; SH; =S; F; Cl; Br; I.

The present invention further provides a method for preparation of the compounds of general formula I.

In preferred method compound of general formula III

T-Z-T (III),

wherein T is halogen, mesyl, tosyl or other leaving group and Z has the meaning as defined above, is subjected to a reaction with trisubstituted phosphine (PR3R4R5), preferably in dimethylformamide (DMF), yielding trisubstituted phosphonium hydrocarbyl derivative of general formula IV

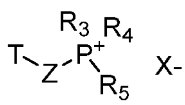
(IV)

which is then condensed with deferoxamine (compound having the structure corresponding to compound I, wherein R1 = R2 = H), preferably in DMF in the presence of base, preferably sodium bicarbonate, yielding trisubstituted phosphonium hydrocarbyl deferoxamine derivative of general formula I.

The compounds of the present invention were tested for their biological effects and compared with the known compound - deferoxamine. The most active compounds of the present invention killed cancer cells with effects higher by 1-2 orders of magnitude than those of deferoxamine. This is unprecedented and very unexpected.

An important finding is that the compounds of the present invention do not show toxic effects on non-malignant cells, hence, they are selective in killing the cancer cells. The compounds of the present invention have a better selectivity index and hence decreased side effects in the treatment.

Furthermore, the compounds of the present invention act by several independent mechanisms which makes them suitable for treatment of various types of resistant cancers. The mechanisms of action include anti-proliferative activity, apoptosis-inducing (or cell death-inducing) activity and anti-migratory activity. These activities are selective to malignant cells. Resistant cancers are typically resistant to medicaments acting through a certain mechanism of action. The compounds of the present invention which have multiple modes of action may then overcome the resistance by using different mode of action to which the cancer cells are sensitive.

The ability of the compounds of the present invention to act through multiple mechanisms (modes) of action also results in their usability for treatment of various types of proliferative diseases, in particular cancers, such as breast, prostate, GIT, hepatic, colorectal, pancreatic, mesothelioma, lung cancers and leukaemias.

Further, object of the present invention is a method of treatment of mammals, including human, in which one or more compounds of general formula I is administered to a subject suffering from proliferative diseases, such as cancer.

Object of the present invention is also a pharmaceutical preparation containing at least one compound of general formula I and at least one pharmaceutical auxiliary substance, such as a carrier, a solvent, a filler, a colorant, a binder, etc.

Additionally, the present invention includes a pharmaceutical preparation comprising at least one compound of formula I and a metal. The metal is preferably selected from transition metals (B-groups of the periodic table, lanthanides, actinides) and metals of IIIA and IVA groups of the periodic table. The metal may be a radionuclide or a metal suitable for use as a diagnostic or therapeutic agent, for example for radiation therapy, biosensing, bioimaging, drug delivery, gene delivery, photodynamic therapy (in particular metals such as lanthane, cerium, praseodyme, neodyme, promethium, samarium, europium, gadolinium, terbium, dysprosium, holmium, erbium, thulium, ytterbium, lutecium, iron, gallium, copper). At least part of the total amount of the metal and at least part of the total amount of compounds of formula I present in the preparation may form a chelate complex.

In one embodiment, the metal is gallium. Gallium may be in the form of a salt, such as gallium chloride or gallium nitrate. At least part of gallium and compounds of formula I present in the preparation may form a chelate complex. Such pharmaceutical preparation has an even higher cell death-inducing activity than the compounds of formula I alone.

Object of the present invention are thus compounds of general formula I or the pharmaceutical preparation comprising at least one compound of formula I and a metal, preferably gallium, for use as medicaments, in particular for use in a method of treatment of proliferative diseases, such as cancer.

Object of the present invention is use of compounds of general formula I or the pharmaceutical preparation comprising at least one compound of formula I and a metal, preferably gallium, for preparation of a medicament for the treatment of proliferative diseases, such as cancer.

Object of the present invention is thus a pharmaceutical preparation comprising at least one compound of formula I and a metal, preferably gallium, for use as a contrast agent, used for diagnostic in particular for use in a method of *in vivo* visualisation of cancer. Such preparation can be visualized by, e.g., PET.

Object of the present invention is at least one compound of formula I and a metal, preferably gallium, for use in the treatment of a proliferative disease such as cancer, or for use as a contrast agent in diagnostic such as *in vivo* visualisation of cancer, wherein the compound of formula I and the metal are administered simultaneously or sequentially.

Furthermore, within the framework of the present invention it was found that the compounds of formula I show synergistic effects when combined with other anti-cancer active ingredients. In particular, the anti-cancer active ingredients for which this synergistic effect was observed include doxorubicin, paclitaxel, cis-platin, fluorouracil.

An "anti-cancer active ingredient" is a substance or a compound which has a cytotoxic effect on cells of cancer cell lines.

Object of the present invention is thus a pharmaceutical preparation comprising at least one compound of formula I and at least one further anti-cancer active ingredient, preferably selected from doxorubicin, paclitaxel, cis-platin, fluorouracil.

Object of the present invention is thus a pharmaceutical preparation comprising at least one compound of formula I and at least one further anti-cancer active ingredient, preferably selected from doxorubicin, paclitaxel, cis-platin and fluorouracil, for use in the treatment of a proliferative disease such as cancer.

Object of the present invention is at least one compound of formula I and at least one further anti-cancer active ingredient, preferably selected from doxorubicin, paclitaxel, cis-platin and fluorouracil, for use in the treatment of a proliferative disease such as cancer, wherein the at least one compound of formula I and the at least one further anti-cancer active ingredient are administered simultaneously or sequentially.

## Examples of carrying out the Invention

### Example 1

Triphenyl(3,14,25-trihydroxy-2,10,13,21,24-pentaoxo-3,9,14,20,25,3,31-hexaazahentetracontan-41-yl) phosphonium chloride

DMF (1 mL) was added to a flask charged with deferoxamine mesylate (50 mg, 0.076 mmol), (10-bromodecyl) triphenylphosphonium bromide (100 mg, 0.178 mmol) and sodium bicarbonate (64 mg, 0.762 mmol). Reaction mixture was stirred and heated to 60°C after 4 hours was the heater turned off and stirring continued 18 hours at room temperature. The mixture was diluted with dichloromethane (10 mL), filtered and concentrated under vacuum. The resulting oil was

triturated with diethylether (10 mL) and precipitate collected. The precipitate was than dissolved in methanol (3 mL), filtered through ion-exchange resin (3.6g, Dowex 2 x 10-$Cl^-$) and concentrated under vacuum. The crude product was submitted to chromatography (10 mL of Silica, chloroform/methanol/ ammonia 100:5:2→100:10:2→ 100:15:2) to give 15 mg of slightly yellow product of the formula 1.

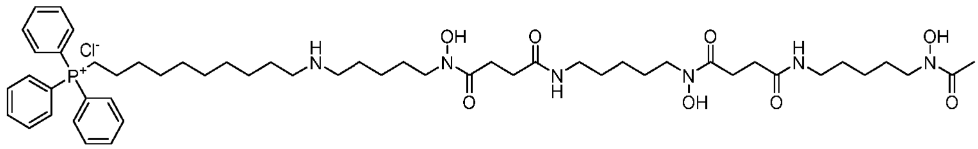

(1)

$R_f$ 0.07 ($CHCl_3$/$CH_3OH$/$NH_3$ 100:10:2);
$^1$H NMR (500 MHz, $CD_3OD$) δ 7.93 - 7.87 (m, 3H), 7.85 - 7.72 (m, 12H), 3.64 - 3.56 (m, 6H), 3.45 - 3.36 (m, 2H), 3.17 (t, *J* = 6.6 Hz, 4H), 2.77 (t, *J* = 7.0 Hz, 4H), 2.60 (dd, *J* = 15.4, 9.2 Hz, 2H), 2.51 - 2.38 (m, 6H), 2.10 (s, 3H), 1.74 - 1.59 (m, 8H), 1.59 - 1.40 (m, 10H), 1.40 - 1.19 (m, 16H).
$^{13}$C NMR (126 MHz, $CD_3OD$) δ 174.90, 174.89, 174.07, 173.99, 172.98, 136.27 (d, *J* = 3.0 Hz), 134.79 (d, *J* = 10.0 Hz), 131.51 (d, *J* = 12.5 Hz), 120.00 (d, *J* = 86.3 Hz), 55.12, 54.70, 50.52, 50.31, 49.84, 40.27 (2C), area of overlaping signals, some of them have *J* coupling with phosphorus - 31.60, 31.55, 31.53, 30.62, 30.54, 30.52, 30.43, 30.37, 30.32, 30.05, 29.98, 29.94, 29.89, 29.85, 29.62, 28.95, 28.91, 28.71, 28.33, 27.43, 27.37, 25.28, 24.95, 24.91, 23.54 (d, *J* = 4.4 Hz), 22.88, 22.47, 20.21.
IR-3400,3303, 3093, 3054, 2927, 2854, 1642, 1622, 1588, 1566, 1481, 1461, 1438, 1373, 1252, 1162, 1113, 996, 746, 723, 691.
HR-MS: m/z = 1, found: 961.59155, calcd. for $C_{53}H_{82}N_6O_8P^+$: 961.59263
HR-MS: m/z = 2, found: 481.29965, calcd. for $C_{53}H_{83}N_6O_8P^{2+}$: 481.29632

Example 2

Triphenyl(3,14,25-trihydroxy-2,10,13,21,24-pentaoxo-31-(10-(triphenylphosphonio)decyl)-3,9,14,20,25,31-hexaazahentetracontan-41-yl)phosphonium chloride

DMF (2 mL) was added to a flask charged with deferoxamine mesylate (100 mg, 0.152 mmol), (10-bromodecyl)triphenylphosphonium bromide (200 mg, 0.356 mmol) and sodium bicarbonate (600 mg, 7.143 mmol). Reaction mixture was stirred and heated to 70°C after 4 hours was the heater turned off and stirring continued 18 hours at room temperature. The mixture was diluted with dichloromethane (20 mL), filtered and concentrated under vacuum. The resulting oil was triturated with diethylether (12 mL) and resulting precipitate again triturated with petrolether (12 mL). Residue was than dissolved in methanol (3 mL), filtered through ion-exchange reisin (7 g, Dowex 2 x 10-$Cl^-$) and concentrated under vacuum. The crude product was submitted to chromatography (10 mL of Silica, chloroform/methanol/ammonia 100:10:1 (200 mL) →100:15:1.5 (200 mL)) to give 48 mg of slightly yellow product of the formula 2.

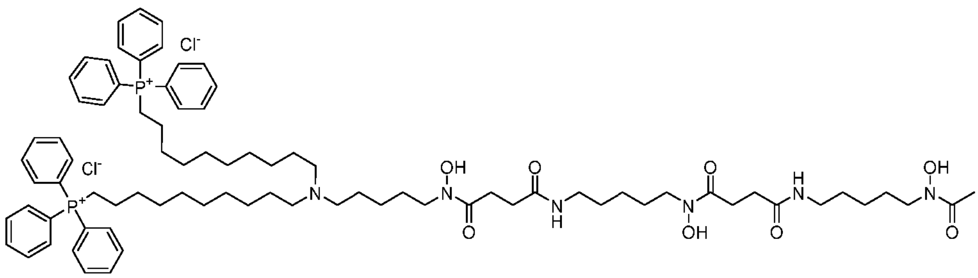

(2)

$R_f$ 0.05 ($CHCl_3$/$CH_3OH$/$NH_3$ 100:10:2);
$^1$H NMR (500 MHz, $CD_3OD$) δ 7.93 - 7.86 (m, 6H), 7.86 - 7.71 (m, 24H), 3.63 - 3.54 (m, 6H), 3.47 - 3.37 (m, 4H), 3.20 - 3.12 (m, 4H), 2.77 (t, *J* = 6.3 Hz, 4H), 2.55 (dd, *J* = 15.9, 8.9 Hz, 4H), 2.46 (t, *J* = 6.8 Hz, 4H), 2.09 (s, 3H), 1.74 - 1.60 (m, 12H), 1.60 - 1.44 (m, 14H), 1.42 - 1.19 (m, 26H).
$^{13}$C NMR (126 MHz, $CD_3OD$) δ 174.8, 174.4, 174.3, 173.3, 136.24 (d, *J* = 3.0 Hz), 134.78 (d, *J* = 10.0 Hz), 131.50 (d, *J* = 12.6 Hz), 119.98 (d, *J* = 86.3 Hz), 55.0, 54.6, 40.2, 31.7-29.8 area of overlaping signals, 31.07 - 29.51 (m), 28.9, 28.5, 27.3, 26.9, 25.5, 24.9 (2C), 23.5(2C), 22.9, 22.4, 20.2.

IR-3264,3059,1636,1588,1547,1485,1457,1439,1362,1259,1114,996,750,729,691 HR-MS: m/z = 2, found: 681.41589, calcd. for $C_{81}H_{116}N_6O_8P^{2+}$: 681.415945
HR-MS: m/z = 3, found: 454.61316, calcd. for $C_{81}H_{117}N_6O_8P^{3+}$: 454.613056

Example 3

Triphenyl(3,14,25-trihydroxy-2,10,13,21,24-pentaoxo-31-(6-(triphenylphosphonio)hexyl)-3,9,14,20,25,31-hexaazaheptatriacontan-37-yl)phosphonium chloride

Deferoxamine mesylate salt (62 mg; 0,094 mmol; 1eq.), (bromohexyl)triphenylphosphonium bromide (400 mg; 0,94 mmol; 10 eq.) and $NaHCO_3$ (372 mg; 4,4 mmol; 47 eq.) were dissolved in dry DMF (2 mL) and heated to 60°C while stirring 4 hours. After that reaction was cooled to rt and stirred additional 18 hours. Reaction progress was checked by TLC ($CHCl_3$/MeOH/$NH_3$; 80/20/2). Reaction was diluted with 60 ml of DCM, $NaHCO_3$ was filtrated off and solvents were evaporated. Product was prescipitated in ice cooled $Et_2O$ (10 ml) and PE (10 ml) and solvents were decanted off. Precipitate was dissolved in methanol (3 ml), filtered through DOWEX (2 x 10 $Cl^-$; 15 g) and concentrated under vacuum. Crude product was purified by column chromatography on silica gel ($CHCl_3$/MeOH/$NH_3$ 100/10/1) which afforded pure orange foam (52 mg, 40 %).

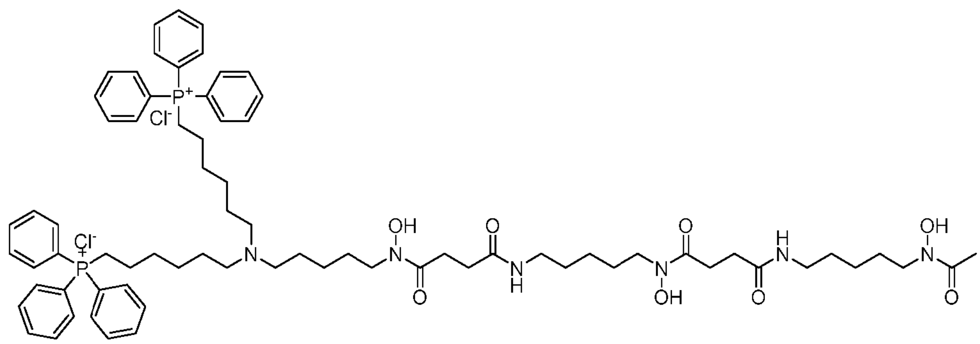

(3)

$^1$H NMR (600 MHz, $CD_3OD$) δ 8.05 - 7.68 (m, 30H), 3.76 - 3.55 (m, 6H), 3.48 (ddt, J = 14.3, 8.1, 3.2 Hz, 4H), 3.25 - 3.08 (m, 8H), 2.83 - 2.74 (m, 4H), 2.48 (q, J = 6.8 Hz, 4H), 2.12 (d, J = 3.9 Hz, 3H), 1.87 - 1.59 (m, 20H), 1.59 - 1.24 (m, 10H).
$^{13}$C NMR (151 MHz, $CD_3OD$) δ 174.78 , 174.61 , 174.46 , 136.30 (d, J = 2.8 Hz), 134.85 (d, J = 10.0 Hz), 131.55 (d, J = 12.6 Hz), 119.94 (d, J = 86.4 Hz), 54.20 , 53.83 , 40.28 , 31.45 (d, J = 11.5 Hz), 31.03 (d, J = 16.7 Hz), 29.98 , 28.90 (d, J = 14.2 Hz), 27.34 , 26.89 , 24.92 , 24.70 , 23.43 (d, J = 4.2 Hz), 22.85 , 22.51 , 20.26.
IR: 3431(s), 3263(m), 3056(m), 2932(m), 2863(m), 1636(s), 1584(m), 1545(m), 1438(s), 1113(s), 996(m), 723(m), 692(m), 532(m), 509(m)
MS: m/z = 2, found: 625.4, calcd. for $C_{73}H_{100}N_6O_8P^{2+}$: 625.35

Example 4

Bromodecyltricyclohexylphosphonium bromide (1,66g mg; 2,9 mmol; 10 eq.), deferoxamine mesylate salt (190 mg; 0,286 mmol; 1eq.) and $NaHCO_3$ (1,13 g; 0,013 mol; 47 eq.) were dissolved in dry DMF and heated to 60°C while stirred for 4h. After that reaction was cooled to rt and stirred overnight. Reaction progress was monitored by TLC ($CHCl_3$/MeOH/$NH_3$; 80/20/2). When finished, reaction was diluted by 20 ml of DCM, $NaHCO_3$ was filtered off and solvents were evaporated. Crude product was dissolved in dichloromethane (5 mL) and precipitated by addition into in ice cooled $Et_2O$ (40 ml) and PE (40 ml). After 1-2 hrs of vigorous stirring was solvent decanted off and resulting precipitate was dissolved in methanol/$H_2O$ (5 ml) and filtrated through DOWEX (45 ml). Solvents were evaporated and product was purified by column chromatography on silica gel ($CHCl_3$/MeOH/$NH_3$ 100/10/1). Reaction afforded yellow oil of bisphosphonium deferoxamine of the formula 4 (148 mg, 35 %) and monophosphonium deferoxamine of the formula 5 (59 mg, 48 %).

Tricyclohexyl(3,14,25-trihydroxy-2,10,13,21,24-pentaoxo-31-(10-(tricyclohexylphosphonio) decyl)-3,9,14,20,25,31-hexaazahentetracontan-31-ium-41-yl) phosphonium trichloride

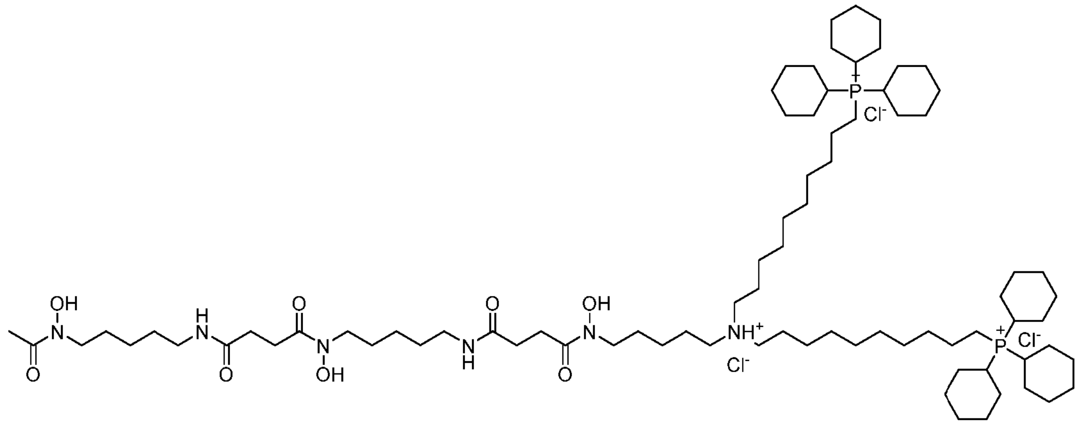

(4)

$^1H$ NMR (500 MHz, Methanol-d4) δ 4.59 (s, 2H), 3.68 - 3.58 (m, 6H), 3.23 - 3.12 (m, 4H), 2.78 (t, J = 7.2 Hz, 6H), 2.61 - 2.44 (m, 8H), 2.31 - 2.20 (m, 4H), 2.11 (s, 3H), 2.07 - 1.88 (m, 22H), 1.87 - 1.76 (m, 6H), 1.72 - 1.33 (m, 44H).
HRMS: Calculated: 699,55680 Found: 699,55727 (m/z = 2)
IR (KBr pellet): ν = 3423, 3250, 2931, 2854, 1448, 1122, 1008, 722

Tricyclohexyl(3,14,25-trihydroxy-2,10,13,21,24-pentaoxo-3,9,14,20,25,31-hexaazahentetracontan-31-ium-41-yl) phosphonium dichloride

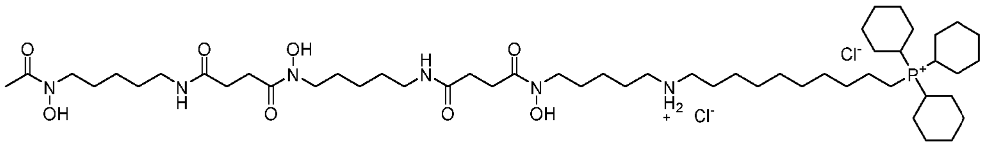

(5)

$^1H$ NMR (500 MHz, Methanol-d4) δ 4.60 (s, 5H)*, 3.63 (dt, J = 17.7, 6.9 Hz, 6H), 3.23 - 3.13 (m, 6H), 3.05 - 2.90 (m, 4H), 2.78 (t, J = 7.1 Hz, 4H), 2.61 - 2.43 (m, 8H), 2.25 (dd, J = 16.9, 12.3 Hz, 2H), 2.11 (s, 3H), 2.04 - 1.27 (m, 67H).
HRMS: Calculated 979,73348 Found 979,73383; Calculated 490,37038 Found 490,37051 (m/z = 2)
IR (KBr pellet): ν = 3059, 2931, 2854, 1641, 1547, 1448, 722

Example 5

42,42-dibutyl-3,14,25-trihydroxy-2,10,13,21,24-pentaoxo-31-(10-(tributylphosphonio)decyl)-3,9,14,20,25,31-hexaaza-42-phosphahexatetracontane-31,42-diium trichloride

Tri(n-butyl)bromodecyl phosphonium bromide (1,45g mg; 2,9 mmol; 10 eq.), deferoxamine mesylate salt (190 mg; 0,286 mmol; 1eq.) and $NaHCO_3$ (1,15 g; 0,014 mol; 47 eq.) were dissolved in dry DMF (20 ml) and heated to 60°C while stirred for 4h. After that reaction was cooled to rt and stirred overnight. Reaction progress was monitored by TLC ($CHCl_3$/MeOH/$NH_3$; 80/20/2). Reaction was diluted with 20 ml of DCM, $NaHCO_3$ was filtrated off and solvents were evaporated. Crude product was dissolved in dichloromethane (5 mL) and precipitated by addition into ice cooled $Et_2O$ (40 ml) and PE (40 ml). After two hours of vigorous stirring was solvent decanted off and precipitate was dissolved in methanol/$H_2O$ (5 ml) and filtered through DOWEX (45 ml). Solvents were evaporated. Product was purified by column chromatography on silica gel ($CHCl_3$/MeOH/$NH_3$ 100/10/1). Reaction afforded yellow oil of the formula 6 (22 mg, 22 %).

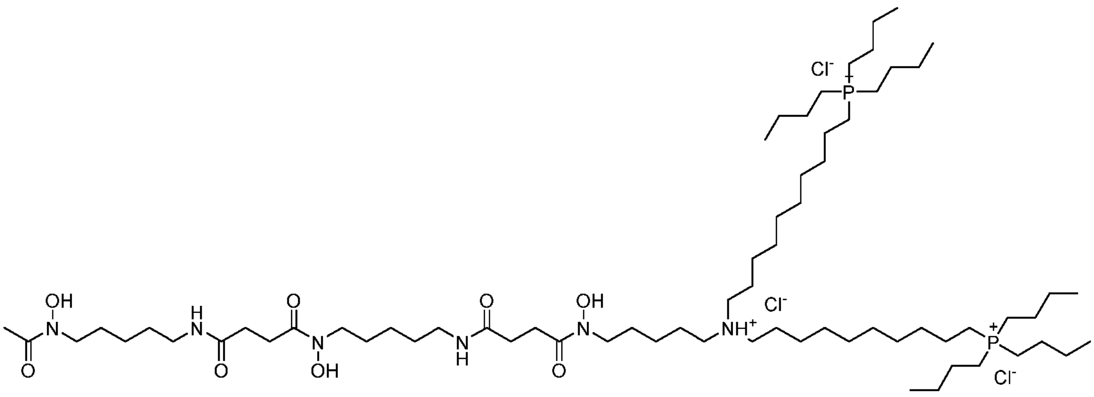

(6)

$^1$H NMR (500 MHz, Methanol-d4) δ 4.59 (s, 2H), 3.70 - 3.56 (m, 6H), 3.24 - 3.13 (m, 6H), 2.83 (s, 6H), 2.78 (t, J = 7.2 Hz, 4H), 2.47 (q, J = 7.0 Hz, 4H), 2.31 - 2.14 (m, 16H), 2.11 (s, 3H), 1.84 - 1.17 (m, 74H), 1.02 (t, J = 7.0 Hz, 18H).
HRMS: Calculated: 621,50985 Found: 621,51044 (m/z = 2)
IR (KBr pellet): v = 3066, 2930, 2857, 1640, 1550, 1101, 720

Example 6

Triphenyl(3,14,25-trihydroxy-2,10,13,21,24-pentaoxo-31-(12-(triphenylphosphonio)dodecyl)-3,9,14,20,25,31-hexaazatritetracontan-31-ium-43-yl)phosphonium trichloride

Triphenylphosphoniumdodecyl bromide (1,5g mg; 2,5 mmol; 10 eq.), deferoxamine mesylate salt (170 mg; 0,25 mmol; 1eq.) and $NaHCO_3$ (1 g; 0,012 mol; 47 eq.) were dissolved in dry DMF (20 ml) and heated to 60°C and stirred at this temperature for 4h. After that reaction was cooled to RT and stirred overnight. Reaction process was monitored by TLC ($CHCl_3$/MeOH/$NH_3$; 80/20/2). Reaction was diluted by 20 ml of DCM, $NaHCO_3$ was filtrated of and solvents were evaporated. Product was dissolved in dichloromethane (5 mL) and precipitated by addition in ice cooled $Et_2O$ (40 ml) and PE (40 ml) and decanted. Precipitate was dissolved in methanol/$H_2O$ (5 ml) and filtrated through DOWEX (45 ml) and solvents were evaporated. Product was purified by column chromatography on silica gel ($CHCl_3$/Me-OH/$NH_3$ 100/15/1,5). Reaction afforded yellow oil of the formula 8 (47 mg, 63 %).

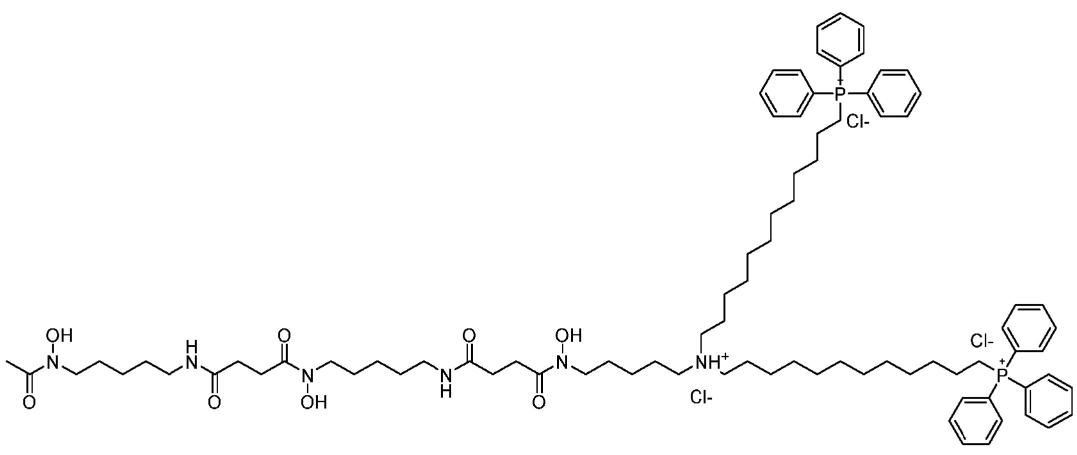

(8)

$^1$H NMR (500 MHz, Methanol-d4) δ 8.02 - 7.66 (m, 30H), 3.61 (t, J = 6.9 Hz, 6H), 3.47 - 3.37 (m, 4H), 3.18 (dt, J = 6.5, 2.2 Hz, 6H), 2.78 (s, 4H), 2.55 (s, 4H), 2.51 - 2.40 (m, 4H), 2.11 (s, 3H), 1.75 - 1.60 (m, 10H), 1.54 (dd, J = 13.2, 5.9 Hz, 10H), 1.40 - 1.18 (m, 34H).
HRMS: Calculated:1418,8945; Found: 709,44728 (m/z = 2)
IR (KBr pellet): v = 2927, 2854, 1636, 1588, 1485, 1439, 1114, 996, 724, 691

Example 7

2,2-dibenzyl-19,30,41-trihydroxy-20,23,31,34,42-pentaoxo-1-phenyl-13-(10-(tribenzylphosphonio)decyl)-13,19,24,30,35,41-hexaaza-2-phosphatritetracontane-2,13-diium trichloride

Tribenzyllphosphoniumdecyl bromide (1,57g mg; 2,6 mmol; 10 eq.), deferoxamine mesylate salt (170 mg; 0,26 mmol; 1eq.) and $NaHCO_3$ (1,03 g; 0,012 mol; 47 eq.) were dissolved in dry DMF (20 ml) and heated to 60°C and stirred at this temperature for 4h. After that reaction was cooled to rt and stirred overnight. Reaction process was monitored by TLC ($CHCl_3$/MeOH/$NH_3$; 80/20/2). Reaction was diluted by 20 ml of DCM, $NaHCO_3$ was filtered off and solvents were evaporated. Crude product was dissolved. Solvents were in dichloromethane (5 mL) and precipitated by addition in ice cooled $Et_2O$ (40 ml) and PE (40 ml) and decanted off and precipitate was dissolved in methanol/$H_2O$ (5 ml) and slowly filtered through DOWEX (25 ml). Solvents were evaporated and product was purified by column chromatography on silica gel ($CHCl_3$/MeOH/$NH_3$ 100/15/1),5. Reaction afforded yellow foam of the formula 9 (52 mg, 71 %).

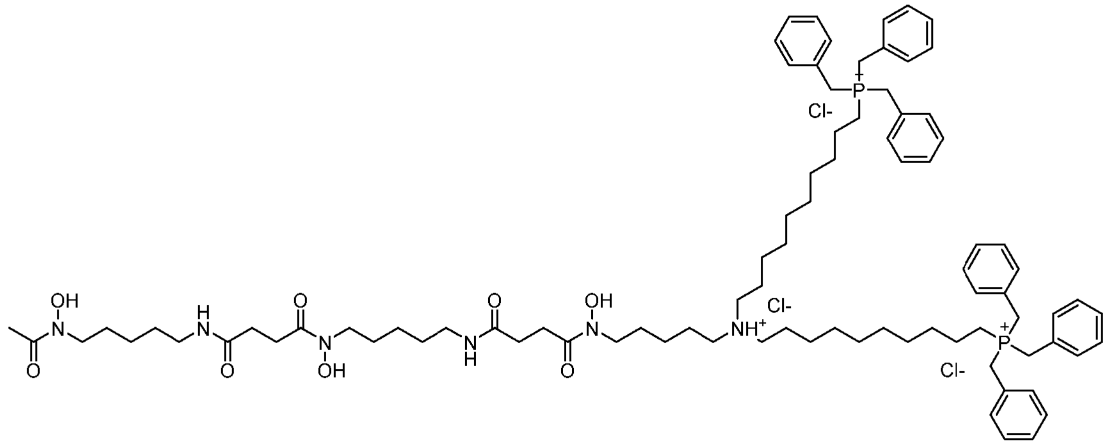

(9)

$^1$H NMR (500 MHz, Methanol-d4) δ 7.52 - 7.21 (m, 30H), 3.80 (dd, J = 14.4, 6.7 Hz, 12H), 3.70 - 3.57 (m, 6H), 3.26 - 3.11 (m, 4H), 2.78 (t, J = 7.2 Hz, 4H), 2.64 (s, 6H), 2.48 (dd, J = 7.1, 3.4 Hz, 4H), 2.11 (s, 3H), 2.04 (d, J = 4.0 Hz, 4H), 1.74 - 1.60 (m, 6H), 1.55 (d, J = 8.2 Hz, 8H), 1.49 - 1.16 (m, 26H).
HRMS: Calculated: 1446,9258; Found:723,46320 (m/z = 2)
IR (KBr pellet): v = 3063, 1636, 1551, 1496, 1455, 1257, 1075, 702.

Example 8

Trioctylbromodccylphosphonium bromide (1,73g mg; 2,6 mmol; 10 cq.), deferoxamine mesylate salt (170 mg; 0,26 mmol; 1eq.) and $NaHCO_3$ (1,03 g; 0,012 mol; 47 eq.) were dissolved in dry DMF (20 ml) and heated to 60°C while stirred for 2,5h. After that reaction was cooled to rt and stirred overnight. Reaction progress was monitored by TLC ($CHCl_3$/MeOH/$NH_3$; 80/20/2). Reaction was diluted by 20 ml of DCM, $NaHCO_3$ was filtered off and solvents were evaporated. Crude product was dissolved in methanol/$H_2O$ (5 ml) and filtered through DOWEX (25 ml) and solvents were evaporated. Product as chloride was purified by column chromatography on silica gel ($CHCl_3$ -$CHCl_3$/MeOH/$NH_3$ 100/15/1,5). Reaction afforded yellow foam of bisphosphonium of the formula 10 (34 mg, 51 %) and 7 mg (5%) of monophosphonium of the formula 11.

3,14,25-trihydroxy-42,42-dioctyl-2,10,13,21,24-pentaoxo-31-(10-(trioctylphosphonio)decyl)-3,9,14,20,25,31-hexaaza-42-phosphapentacontane-31,42-diium trichloride

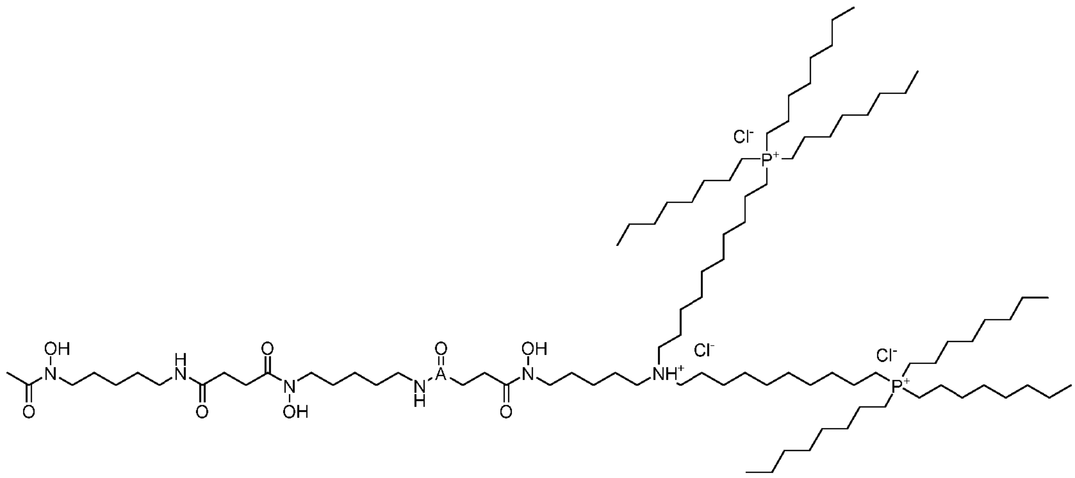

(10)

$^1H$ NMR (500 MHz, Methanol-d4) δ 3.67 - 3.58 (m, 6H), 3.56 (dt, J = 7.8, 6.5 Hz, 4H), 3.21 - 3.15 (m, 6H), 2.78 (t, J = 7.2 Hz, 4H), 2.48 (dd, J = 7.5, 4.9 Hz, 4H), 2.22 (ddd, J = 16.7, 10.4, 6.7 Hz, 16H), 2.11 (s, 3H), 1.83 - 1.70 (m, 10H), 1.63 - 1.32 (m, 120H), 0.92 (t, J = 6.6 Hz, 18H).
HRMS: Calculated: 790.20129; Found:790.20123 (M/Z=2)
IR (KBr pellet): ν = 3069, 2927, 1641, 1544, 1461, 723.

3,14,25-trihydroxy-42,42-dioctyl-2,10,13,21,24-pentaoxo-3,9,14,20,25,31-hexaaza-42-phosphapentacontane-31,42-diium dichloride

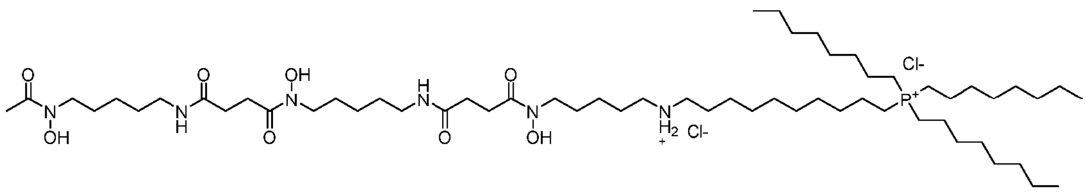

(11)

$^1H$ NMR (500 MHz, Methanol-d4) δ 3.69 - 3.60 (m, 6H), 3.23 - 3.13 (m, 4H), 3.01 (t, J = 7.9 Hz, 4H), 2.79 (t, J = 7.1 Hz, 4H), 2.48 (q, J = 7.1 Hz, 4H), 2.33 - 2.16 (m, 8H), 2.12 (s, 3H), 1.72 - 1.26 (m, 70H), 0.93 (t, J = 6.5 Hz, 9H).
HRMS: Calculated: 535,44080; Found:535,44135 (m/z = 2)
IR (KBr pellet): ν = 3116 (s), 2928 (s), 2856 (m), 2331 (m), 1641 (m), 1558 (m), 724 (m).

Example 9

13-(10-(dimethyl(phenyl)phosphonio)decyl)-19,30,41-trihydroxy-2-methyl-20,23,31,34,42-pentaoxo-2-phenyl-13,19,24,30,35,41-hexaaza-2-phosphatritetracontan-2-ium trichloride

Dimethylphenylbromodecylphosphonium bromide (1,3g mg; 2,9 mmol; 10 eq.), deferoxamine mesylate salt (190 mg; 0,30 mmol; leq.) and $NaHCO_3$ (1,17 g; 0,014 mol; 47 eq.) were dissolved in dry DMF (20 ml) and heated to 60°C while stirred 4h. After that reaction was cooled to RT and stirred overnight. Reaction progress was monitored by TLC ($CHCl_3$/MeOH/$NH_3$; 80/20/2).

Reaction was diluted by 20 ml of DCM, $NaHCO_3$ was filtered off and solvents were evaporated. Crude product was dissolved in methanol/$H_2O$ (5 ml) and filtered through DOWEX (25 ml). All solvents were evaporated and product as chloride was purified by column chromatography on silica gel ($CHCl_3$ -$CHCl_3$/MeOH/$NH_3$ 100/15/1,5). Reaction afforded yellow foam of the structure 12 (54 mg, 57 %).

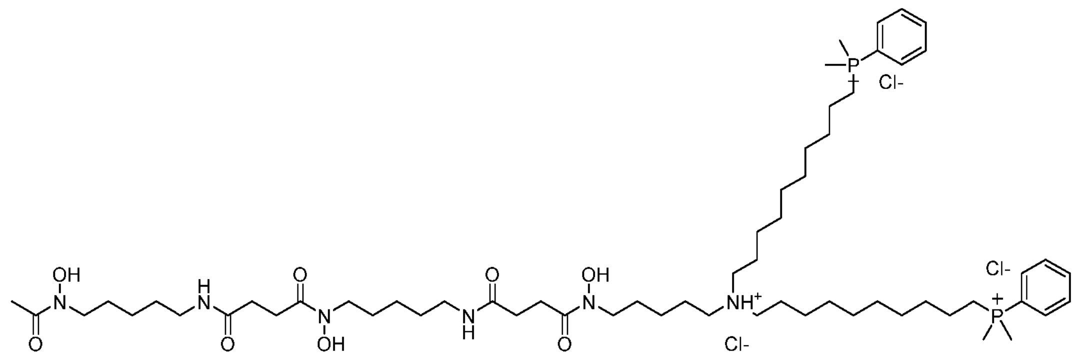

(12)

$^1$H NMR (500 MHz, Methanol-d4) δ 8.04 - 7.67 (m, 10H), 3.62 (qd, J = 8.1, 6.9, 4.7 Hz, 6H), 3.23 - 3.15 (m, 4H), 3.04 (s, 6H), 2.78 (s, 4H), 2.57 - 2.44 (m, 12H), 2.24 (dd, J = 12.7, 3.1 Hz, 12H), 2.11 (s, 3H), 1.75 - 1.27 (m, 40H).
HRMS: Calculated: 557,38465; Found: 557,38488 (m/z = 3)
IR (KBr pellet): ν = 3434 (m), 3260 (m), 3063 (w), 2928 (s), 2856 (m), 1638 (s), 1548 (m), 1457 (m), 1438 (m), 1122 (m), 998 (m), 749 (m), 692 (m).

Example 10

(10-(4-hydroxybutoxy)decyl)triphenylphosphonium bromide

Butan-1,4-diol (1g; 0,011 mol) and NaH (480 mg; 0,012 mol) were dissolved in DMF (10 ml) and stirred at rt 15 minutes. After that triphenylphosphoniumbromodecyl bromide (7,7 g; 0,014 mol) in DMF (20 ml) was added dropwise and reaction mixture was stirred at rt 1 hour. Reaction process was monitored by TLC ($CHCl_3$/MeOH 10:1). Reaction was washed between 5 mL of water and DCM (3 x 10 mL). Column chromatography on silicagel ($CHCl_3$/MeOH 0-10%) afforded product as yellow oil of the structure 13 (1,5 g; 24%).

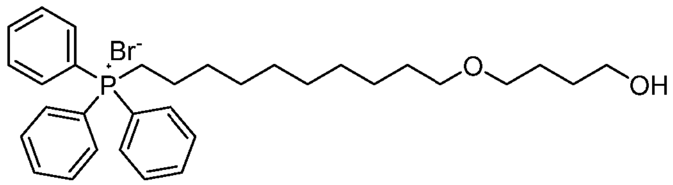

(13)

$^1$H NMR (500 MHz, Methanol-d4) δ 7.99 - 7.69 (m, 15H), 3.58 (t, J = 6.2 Hz, 2H), 3.45 (dt, J = 13.2, 6.2 Hz, 4H), 1.82 - 1.43 (m, 12H), 1.31 (d, J = 9.1 Hz, 10H).
HRMS: Calculated: 491,30734; Found: 491,30750 (m/z = 2)
IR (KBr pellet): ν = 3078 (w), 3054 (w), 3008 (w), 2928 (s), 2855 (s), 1587 (m), 1576 (m), 1485 (m), 1465 (m), 1438 (s), 1373 (m), 1114 (s), 1058 (m), 996 (m), 750 (m), 723 (m), 691 (m).

Example 11

(10-(4-bromobutoxy)decyl)triphenylphosphonium bromide

(10-(4-hydroxybutoxy)decyl)triphenylphosphonium bromide (0,5 g; 0,87 mmol), tetrabromomethane (0,75 g; 2,2 mmol) and triphenylphosphine (0,68 g; 2,6 mmol) were dissolved in DCM (10 ml) and stirred at rt overnight. Reaction was monitored by TLC ($CHCl_3$/MeOH 10:1). Reaction was quenched by addition of saturated aqueous $NaHCO_3$ and extracted with DCM (30 mL). Column chromatography on silicagel ($CHCl_3$/MeOH 25:1) afforded yellow oil of the structure 14 (280 mg; 50%).

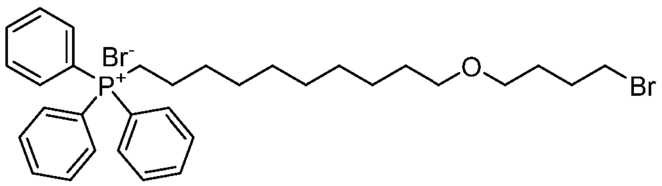

(14)

$^1H$ NMR (500 MHz, Methanol-d4) δ 8.08 - 7.57 (m, 15H), 3.47 (dt, J = 7.7, 6.5 Hz, 4H), 3.43 (t, J = 6.5 Hz, 2H), 1.93 (p, J = 6.9 Hz, 2H), 1.77 - 1.64 (m, 4H), 1.62 - 1.51 (m, 4H), 1.42 - 1.21 (m, 12H).
HRMS: Calculated: 553,22294; Found: 553,22316
IR (KBr pellet): ν = 3053 (m), 3006 (w), 1927 (s), 1854 (s), 1587 (m), 1485 (m), 1465 (m), 1438 (s), 1114 (s), 996 (m), 750 (m), 723 (m), 691 (m).

Example 12

3-(diphenyl(3,14,25-trihydroxy-2,10,13,21,24-pentaoxo-31-(4-((10-(triphenylphosphonio) decyl)oxy)butyl)-36-oxa-3,9,14,20,25,31-hexaazahexatetracontan-31-ium-46-yl) phosphonio)benzen-1-ide trichloride

(10-(4-bromobutoxy)decyl)triphenylphosphonium bromide (1,14g mg; 1,8 mmol; 10 eq.), deferoxamine mesylate salt (118 mg; 0,18 mmol; 1eq.) and $NaHCO_3$ (711 mg; 8,46 mmol; 47 eq.) were dissolved in dry DMF and heated to 60°C while stirred for 4h. After that reaction was cooled to rt and stirred overnight. Reaction progress was monitored by TLC ($CHCl_3$/MeOH/$NH_3$; 80/20/2). Reaction was diluted by 20 ml of DCM, $NaHCO_3$ was filtered off and solvents were evaporated. Product was filtered through DOWEX (45 ml) and solvents were evaporated. Product was purified by column chromatography on silica gel ($CHCl_3$/MeOH/$NH_3$ 100/10/1). Reaction afforded light yellow foam of the formula 15 (155 mg, 55 %).

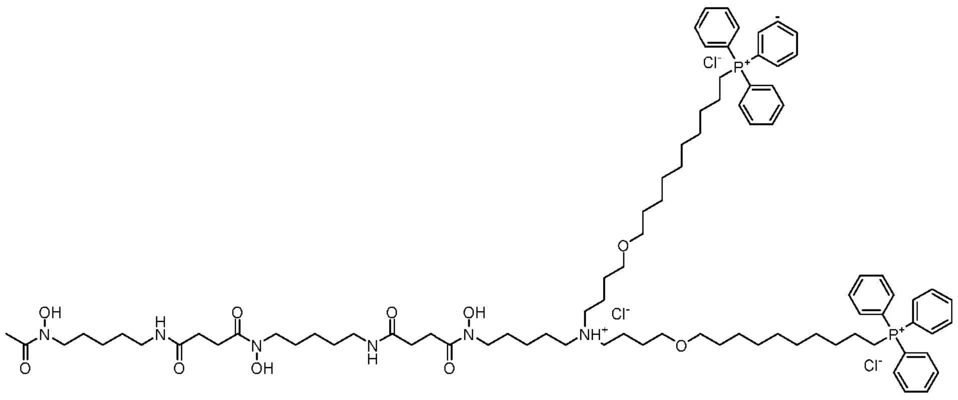

(15)

$^1H$ NMR (500 MHz, Methanol-d4) δ 7.99 - 7.65 (m, 30H), 3.73 - 3.55 (m, 8H), 3.57 - 3.37 (m, 14H), 3.18 (tt, J = 6.9, 2.9 Hz, 4H), 2.78 (q, J = 4.8, 2.9 Hz, 4H), 2.60 (s, 6H), 2.47 (t, J = 7.2 Hz, 4H), 2.11 (d, J = 1.9 Hz, 3H), 1.74 - 1.20 (m, 58H).
HRMS: Calculated: 753,47346; Found: 753,47410 (m/z = 2)
IR (KBr pellet): ν = 3411(m), 3257 (m), 3058 (m), 2929 (s), 2855 (s), 1640 (s), 1588 (m), 1485 (m), 1460 (m), 1439 (s), 1370 (m), 1114 (s), 1045 (m), 996 (m), 748 (m), 724 (m), 691 (m).

Example 13

(15,26-diacetoxy-4-acetyl-2,11,14,22,25-pentaoxo-32-(10-(triphenylphosphonio)decyl)-3-oxa-4,10,15,21,26,32-hexaazadotetracontan-32-ium-42-yl)triphenylphosphonium trichloride

Compound 2 (20 mg; 0,0139 mmol) was dissolved in DCM (1 ml) and cooled to 4°C, $Ac_2O$ (0,024 ml; 0,250 mmol) and pyridine (0,01 ml; 0,125 mmol) were added. Reaction was stirred 1 hour at 4°C and then allowed to rt. TLC analysis ($CHCl_3$/MeOH 5:1) indicated full conversion of starting material. Reaction mixture was cooled to 4°C and 5 ml $Et_2O$ was added. Product slowly precipitated off as an oil and solvent was decanted off. Precipitate was dissolved in DCM (3 mL) and residual solvents were evaporated under vacuum. Reaction afforded 20 mg (90%) of essentially pure product of the formula 16.

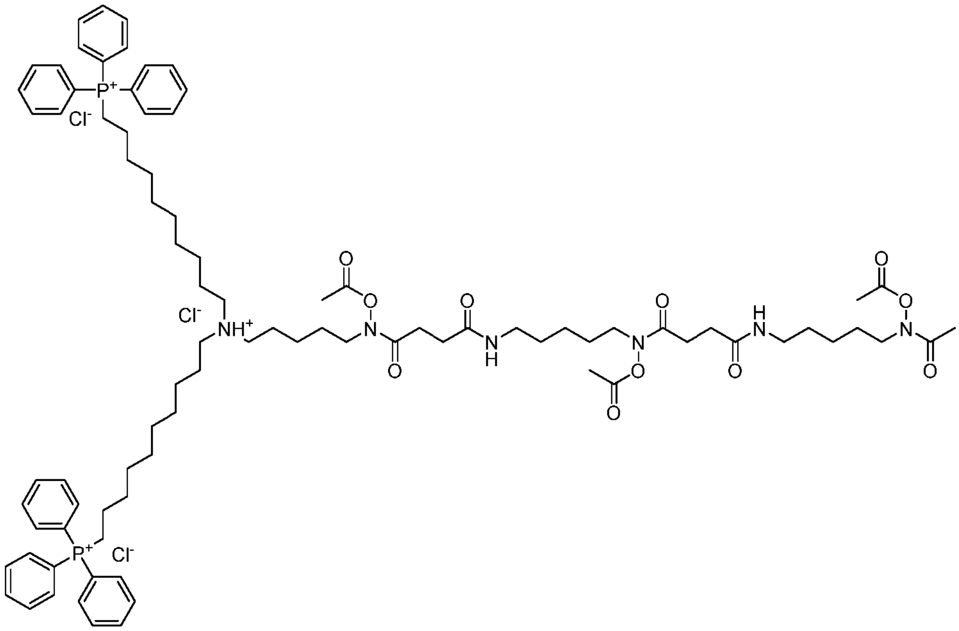

(16)

$^1$H NMR (500 MHz, Methanol-d4) δ 7.97 - 7.69 (m, 30H), 3.70 (td, J = 20.1, 17.8, 9.8 Hz, 6H), 3.48 - 3.38 (m, 4H), 3.23 - 3.11 (m, 4H), 3.12 - 3.00 (m, 4H), 2.59 (s, 4H), 2.48 (t, J = 7.1 Hz, 4H), 2.24 (s, 9H), 1.95 (s, 3H), 1.79 - 1.45 (m, 26H), 1.47 - 1.25 (m, 24H).
HRMS: Calculated 744,43179; Found 744,43214 (m/z = 2)
IR (KBr pellet): v = 3058 (m), 2928 (s), 2854 (s), 1791 (m), 1653 (s), 1558 (m), 1457(m), 1440(m), 1111(m), 990(m), 724(m), 691(m).

Example 14

(39-acetyl-18,21,29,32,41-pentaoxo-17,28-bis(palmitoyloxy)-11-(10-(triphenylphosphonio) decyl)-40-oxa-11,17,22,28,33,39-hexaazahexapentacontyl)triphenylphosphonium trichloride

Compound 2 (20 mg; 0,0139 mmol) was dissolved in DCM (1 ml) and cooled to 4°C, palmitoyl chloride (0,08 ml; 0,25mmol) and pyridine (0,01 ml; 0,125 mmol) were added. Reaction was stirred 1 hour at 4°C and then allowed to rt. TLC analysis ($CHCl_3$/MeOH 5:1) indicated full conversion of starting material. Reaction mixture was cooled to 4°C and 5 ml $Et_2O$ was added. Product slowly precipitated off as an oil and solvent was decanted off. Precipitation of product (1 mL dichloromethane + 5 mL $Et_2O$ at 4°C) was repeated six times. Final precipitate was dissolved in DCM (3 mL) and residual solvents were evaporated under vacuum. Reaction afforded 20 mg (67 %) of essentially pure product of the formula 17.

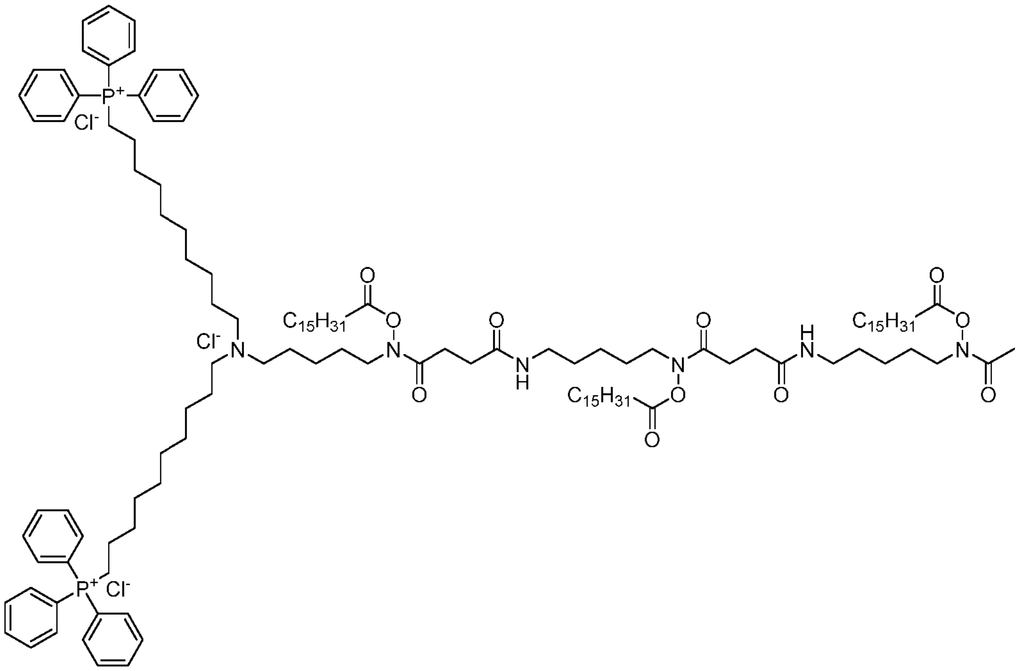

(17)

$^1H$ NMR (500 MHz, Methanol-d4) δ 8.04 - 7.65 (m, 30H), 3.70 (t, J = 21.5 Hz, 4H), 3.43 (dtd, J = 16.3, 8.5, 4.9 Hz, 4H), 3.24 - 3.08 (m, 10H), 2.51 (d, J = 32.4 Hz, 10H), 1.99 (s, 3H), 1.81 - 1.19 (m, 128H), 0.92 (t, J = 6.8 Hz, 9H).
HRMS: Calculated: 1038,76044; Found: 1038,76068
IR (KBr pellet): ν = 3081(m), 3057(m), 2925(s), 2854(s), 1786(m), 16663(s), 1588(m), 1560(m)m, 1484(m), 1465(m), 1457(m), 1439(m), 1114(m), 1081(m), 996(m), 750(m), 724(m), 692(m).

Example 15

(10-azidodecyl)triphenylphosphonium bromide

(10-bromodecyl)triphenylphosphonium bromide (500 mg, 0.889 mmol) was dissolved in DMF (15 mL) and sodium azide (575 mg, 8.845 mmol) was added in one portion under stirring. Mixture was heated under 80°C overnight. Reaction was monitored with TLC (PMA stain, chloroform/ methanol/ammonium 80/20/2, Rf of starting material is the same as of the product 0-0.2, but slightly different color occurs during heating the plate). Sodium azide was filtered off after cooling to laboratory temperature, DMF was evaporated and crude material was purified with short column of silicagel (eluent: chloroform/methanol 10/1). Product was obtained in the form of yellowish oil 450 mg (96 %) of the formula 18.

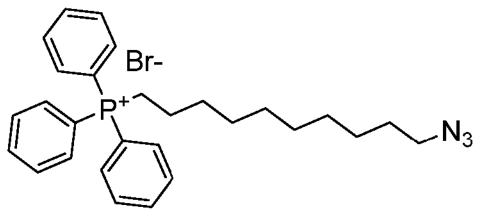

(18)

$^1H$ NMR (500 MHz, Methanol-d4) δ 7.98 - 7.76 (m, 15H), 3.46 (ddd, J = 16.4, 8.2, 5.4 Hz, 2H), 3.30 (t, J = 6.8 Hz, 2H), 1.71 (m, 2H), 1.60 (m, 4H), 1.46 - 1.22 (m, 10H).
HRMS calcd for C28H35N3P 444.25631 found: 444.25643.
IR (KBr pellet): ν = 3395, 3052, 2926, 2854, 2094, 2003, 1587, 1485, 1438, 1345, 1256, 1190, 1162, 1113, 996, 790, 751, 723, 691, 616, 533, 509.

Example 16

N1-(5-(di(prop-2-yn-1-yl)amino)pentyl)-N1-hydroxy-N4-(5-(N-hydroxy-4-((5-(N-hydroxy acetamido)pentyl)amino)-4-oxobutanamido)pentyl)succinamide

Deferoxamine mesylate (100 mg, 0.1523 mmol) was dissolved in DMF (2 mL) and $NaHCO_3$ (384 mg, 4.571 mmol) was added in one portion followed with propargyl bromide (33 μl, 0.306 mmol) as 80% solution in toluene. Reaction mixture was stirred under 80°C for 4 hours and monitored with TLC (PMA stain, chloroform/ methanol/ammonium 80/20/2, Rf 0.45). When reaction was completed reaction mixture was allowed to cool to laboratory temperature, dichloromethane (5 ml) was added and reaction mixture was filtered, evaporated and dried. Crude product was purified with column of silicagel (eluent: chloroform/ methanol/ammonium 80/20/2) to get product in the form of white/yellow solid 69 mg (71%) of the formula 19.

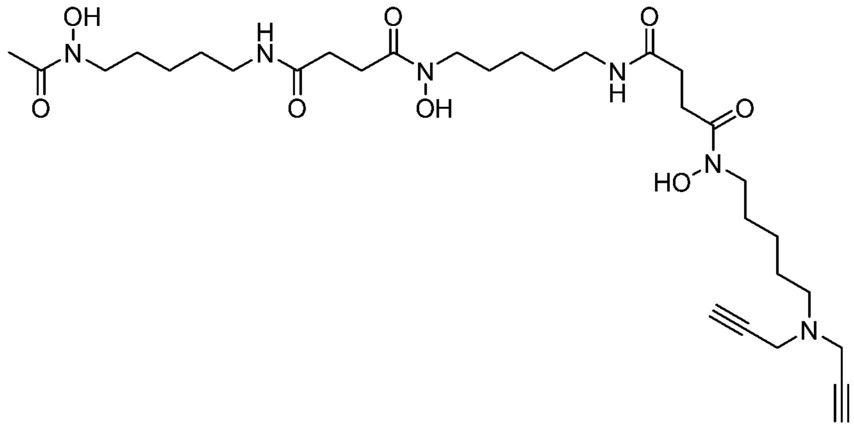

(19)

$^1$H NMR (500 MHz, Methanol-d4) δ 4.58 (s, 2H), 3.60 (t, J = 7.0 Hz, 6H), 3.44 (s, 3H), 3.43 (s, 2H), 3.17 (t, J = 7.0 Hz, 4H), 2.77 (t, J = 7.2 Hz, 4H), 2.63 (t, J = 2.4 Hz, 2H), 2.58 - 2.52 (m, 2H), 2.49 - 2.42 (m, 4H), 2.09 (s, 3H), 1.71 - 1.59 (m, 6H), 1.58 - 1.47 (m, 7H), 1.40 - 1.27 (m, 8H). HRMS calcd for C31H53O8N6 637.39194 found: 637.39240, calcd for C31H53O8N6Na 659.37388 found: 659.37393.

IR (KBr pellet): ν = 3323, 3290, 3143, 2929, 2857, 2113, 1654, 1623, 1565, 1457, 1268, 1253, 1192,1159,960,726,676.

Example 17

Triphenyl(10-(5-(8,19,30-trihydroxy-9,12,20,23,31-pentaoxo-2-((1-(10-(triphenylphosphonio) decyl)-1H-1,2,3-triazol-5-yl)methyl)-2,8,13,19,24,30-hexaazadotriacontyl)-1H-1,2,3-triazol-1-yl)decyl)phosphonium

Bispropargyldeferoxamine 19 (60mg, 0.094 mmol) together with sodium ascorbate (4 mg, 0.020 mmol) and CuSO4.5H2O (10 mg, 0.040 mmol) was placed in flask and (10-azidodecyl) triphenylphosphonium bromide (100 mg, 0.190 mmol) dissolved in DMF (1mL) and water (1mL) was added. Reaction mixture was stirred under 60°C for 1 hour. Reaction was monitored with TLC (PMA stain, chloroform/ methanol/ ammonium 80/20/2, Rf 0.1). When the reaction was completed, solvents were evaporated, crude material dried and purified with column of silicagel (eluent: chloroform/ methanol/ammonium 80/20/2) to get product in the form of light brown oil 138 mg (87%) of the formula 20.

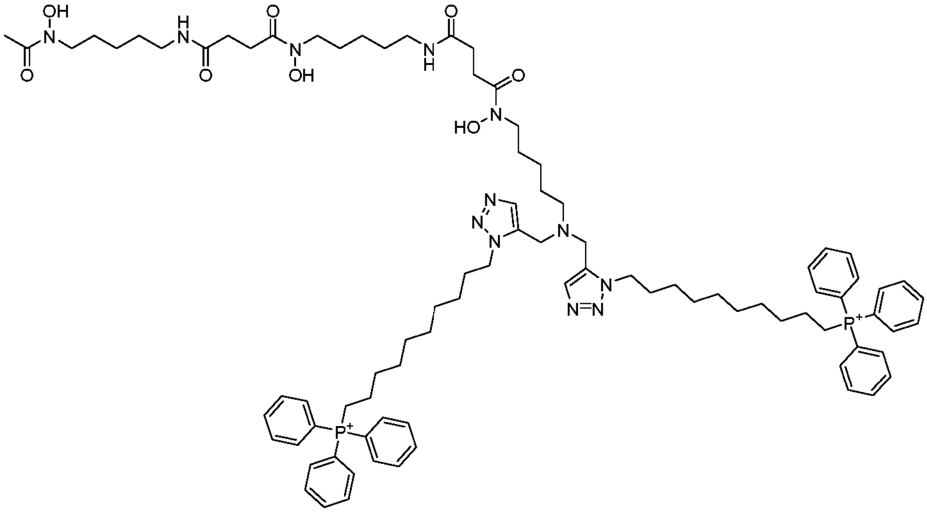

(20)

$^1H$ NMR (500 MHz, Methanol-d4) δ 8.05 (s, 2H), 7.94 - 7.75 (m, 30H), 4.43 (t, J = 6.9 Hz, 4H), 3.87 (s, 4H), 3.66 - 3.54 (m, 6H), 3.49 - 3.40 (m, 4H), 3.24 - 3.13 (m, 4H), 2.83 - 2.74 (m, 4H), 2.52 (m, 2H), 2.50 - 2.44 (m, 4H), 2.12 (s, 3H), 1.92 (t, J = 7.2 Hz, 4H), 1.73 - 1.60 (m, 10H), 1.60 - 1.48 (m, 10H), 1.41 - 1.21 (m, 20H).

HRMS calcd for C87H122O8N12P2 (z = 2) 762.44864 found: 762.44881

IR (KBr pellet): v = 3421, 3259, 2927, 2855, 2212, 1636, 1586, 1546, 1483, 1458, 1438, 1416, 1319, 1257,1213, 1193, 1160, 1113,1052, 1025, 996,792, 750, 723,690, 531,509, 414.

Example 18

Compound 1 and compound 2 were tested in their efficacy to kill malignant MCF7 cancer cells. Briefly, 10.000 cells per well were seeded in a 96-well plat on one day and the next day selected compounds were added and incubated with the cells for 48hrs. The cells were then fixed with 4% paraformaldehyde, stained with 0.05% crystal violet, washed with PBS, and solubilized in 1% SDS. The absorbance of the plate at 595 nm was then measured, quantifying the number of viable cells. Our data show that the compound 2 was more effective and was further tested. Both compounds showed markedly increased efficacy compared to parental deferoxamine (DFO). The comparison is shown in Table 1.

Table 1: The effect of DFO, compound 1 and compound 2 on cellular viability in malignant MCF7 cells after 48hrs of incubation as measured with crystal violet assay that combines cytostatic as well as cytotoxic effect. Data represent mean percentages of viable cells compared to non-treated controls while numbers in brackets represent standard deviations.

| Concentration | Compound 1 | Compound 2 | DFO |
|---|---|---|---|
| 0 μM | 100 (10.8) | 100 (8.6) | 100 (4.1) |
| 1 μM | 83.3 (5.6) | 56.9 (6.6) | -- |
| 2 μM | 48.2 (4.2) | 20.0 (2.1) | 92 (3.0) |
| 5 μM | 15.2 (1.0) | 10.0 (2.7) | 95.9 (3.8) |
| 10 μM | 11.9 (1.2) | 12.1 (1.4) | 92.2 (4.0) |
| $IC_{50}$ (μM) | 2.2 (0.75) | 1.2 (0.3) | >10 |

Example 19

Compound 2 shows ability to kill cancer cells (MCF7 and T47D) while sparing non-malignant cells (BJ), as documented in Table 1 depicting crystal violet staining performed as in example 18 (measuring the combined anti proliferative and cell death-inducing effect) of cells treated with both DFO and compound 2. Importantly, the *in vitro* $IC_{50}$ values of our newly synthesized compound 2 are almost two orders of magnitude lower as compared to the parental DFO, suggesting that it is a highly active compound (Table 1, 2). Also, the compound does show significantly lower $IC_{50}$ values in the malignant cells (MCF7, T47D, MDA-MB-231, BT474) compared to non-malignat BJ fibroblasts. (Table 2a,

2b, 3)

A number of compounds of the present invention were tested for cytotoxicity towards at least some of human breast cancer cell lines (MCF7, MDA-MB-231), human fibroblast (BJ) and murine triple negative breast cancer cell line 4T1 (Table 2).

Table 2a: The effect of DFO and compounds of the present invention on cellular viability in malignant (MCF7, T47D, MDA-MB-231, BT474, 4T1) and non malignant cells (BJ) after 48 hours of incubation, as measured with crystal violet assay. Data represent mean $IC_{50}$ values while numbers in brackets represent standard deviations.

| $IC_{50}$ (μM) | MCF7 | T47D | MDA-MB-231 | BT474 | 4T1 | BJ |
|---|---|---|---|---|---|---|
| DFO | 127.6 (12.5) | 272.4 (23.7) | -- | -- | | 219.1 (20.8) |
| Compound 2 | 1.2 (0.3) | 3.0 (0.7) | 5.4 (0.9) | 3.3 (0.6) | 2.0(0.1) | 14.3 (4.2) |
| Compound 4 | 0.7 (0.1) | | 1.3 (0.2) | | 1.8 (0.1) | |
| Compound 6 | 3.0 (0.3) | | 2.8 (0.5) | | 4.9 (0.3) | |
| Compound 8 | 0.5 (0.1) | | 0.6 (0.1) | | 1.2 (0.1) | |
| Compound 9 | 0.9 (0.1) | | 0.8 (0.1) | | 1.8 (0.1) | |
| Compound 10 | 0.8 (0.1) | | 0.7 (0.1) | | 1.3 (0.1) | |
| Compound 11 | 4.0 (0.3) | | | | | |
| Compound 15 | 0.5 (0.1) | | | | | |
| Compound 16 | 0.8 (0.1) | | | | | |
| Compound 17 | 0.7 (0.1) | | | | | |
| Compound 20 | | | | | 2.2 (0.2) | |

Table 2b: The effect of compound 2 on cellular viability in malignant ovarian and pancreatic cells (NIH:OVCAR-3, SK-OV-3, AsPC-1, BxPC-3, CFPAC-1, PaTu 8902) and non malignant cells (BJ) after 48 hours of incubation, as measured with crystal violet assay. Data represent mean $IC_{50}$ values while numbers in brackets represent standard deviations.

| $IC_{50}$ (μM) | NIH:OVCAR-3 | SK-OV-3 | AsPC-1 | BxPC-3 | CFPAC-1 | PaTu 8902 | BJ |
|---|---|---|---|---|---|---|---|
| Compound 2 | 3.7 (1.6) | 5.1 (1.2) | 2.6 (0.5) | 1.7 (0.4) | 6.9 (1.6) | 5.5 (0.4) | 14.3 (4.2) |

Table 3: Selectivity index for compound 2 ($IC_{50}$ non-malignant/$IC_{50}$ malignant) in MCF7, T47D, MDA-MB-231 and BT474 cells after 48 hours of incubation, as measured with crystal violet assay performed as in example 18.

| Selectivity index | MCF7 | T47D | MDA-MB-231 | BT474 | BJ |
|---|---|---|---|---|---|
| DFO | 1.72 | 0.80 | - | - | 1 |
| Compound 2 | 12.43 | 4.77 | 2.64 | 4.33 | 1 |

Example 20

To test whether compound 2 is active against resistant cancer cells, we used tamoxifen-resistant MCF7, T47D and BT474 cell lines. The $IC_{50}$ values were again measured with the crystal violet assay performed as in example 18. We have documented that there is no statistically significant difference in the ability of compound 2 to induce cytostatic and cytotoxic effects between the parental cells sensitive to tamoxifen and the resistant ones that grow even in the presence of tamoxifen (Table 4). This observation, together with the data suggesting that it is effective also in the triple negative breast cancer cell lines such as MBA-MB-231 and BT549, show that compound 2 is active even in the hard-to-treat cancer subtypes.

Table 4: The effect of compound 2 on cell death induction in malignant (MCF7, T47D, BT474) cells (parental) and their tamoxifen resistant counterparts (Tam5R) grown in the presence of 5 μM tamoxifen. Data represent mean $IC_{50}$ values obtained by the crystal violet assay while numbers in brackets represent standard deviations

| | MCF7 | | T47D | | BT474 | |
|---|---|---|---|---|---|---|
| | Parental | Tam5R | Parental | Tam5R | Parental | Tam5R |
| $IC_{50}$ (μM) | 1.5 (0.2) | 2.1 (0.3) | 3.0 (0.7) | 1.2 (0.3) | 3.3 (0.6) | 4.2 (1.1) |

Example 21

Compound 2 shows efficacy and selectivity as seen in previous tables. To confirm such findings by an independent method, cell death-inducing potency (cytotoxic efficacy) of DFO and compound 2 have been tested *via* annexin V/PI staining commonly used as a measure of apoptotic/necrotic cell death. Briefly, cells were seeded in 12-well plate at 100,000 per well, incubated with the selected compounds for 48 hours, then floating cells were spun, adherent cells trypsinized and spun as well followed by the staining with AnnexinV-FITC and PI probes. Cells were then washed with PBS and measured via Fluorescence Activated Sorter. Cells exhibiting AnnexinV and/or PI positivity are considered as dead cells. As shown in Table 5, compound 2 exhibits a markedly enhanced capacity to induce cell death as compared to DFO in all malignant breast cancer cells. Importantly, non-malignant BJ cells were significantly less sensitive to the effect of compound 2 and toxic effect could be detected only in concentrations higher than 20μM where all tested cancer cells exhibited profound proportion of dead cells (Table 5). This also shows that the effect on cellular viability on crystal violet staining for BJ cells is rather proliferation inhibition (cytostatic effect) while in other malignant cells it is a combined effect of proliferation inhibition and cell death induction, unless high doses over 20 μM were used where it starts to be non-selective.

Table 5: The effect of compound 2 and DFO on cell death induction in malignant (MCF7, T47D, MDA-MB-231, BT549, BT474) and non malignant cells (BJ) as measured with AnnexinV/PI assay after 48 hrs. Data represent mean percentage of dead cells while numbers in brackets represent standard deviations.

| Concentration | MCF7 | T47D | BT474 | MDA-MB-231 | BT549 | BJ |
|---|---|---|---|---|---|---|
| 0 μM | 7.3 (1.2) | 11.0 (1.5) | 5.2 (2.2) | 6.6 (1.1) | 9.3 (1.3) | 1.8 (0.2) |
| DFO 200 μM | 45.0 (14.9) | 14.1 (7.2) | 19.2 (1.4) | 22.4 (3.6) | 18.3 (9.8) | 10.5 (2.2) |
| Compound 2 5 μM | 35.6 (8.1) | 38.3 (5.8) | 18.6 (7.2) | 39.8 (9.7) | 31.8 (6.0) | 6.7 (1.7) |
| Compound 2 10 μM | 43.7 (6.0) | 41.2 (3.1) | 39.9 (23.7) | 78.5 (5.1) | 52.3 (3.2) | 9.0 (3.5) |
| Compound 2 20 μM | 64.0 (9.8) | 93.6 (1.3) | 92.5 (9.3) | 98.0 (1.5) | 80.4 (4.3) | 29.8 (4.8) |

Example 22

Application of compound 2 leads to marked inhibition of cellular respiration as evidenced by two independent methods: the Oxygraph instrument (Rohlenova,K., Sachaphibulkij,K., Stursa,J., Bezawork-Geleta,A., Blecha,J., Endaya,B., Werner,L., Cerny,J., Zobalova,R., Goodwin,J., Spacek,T., Alizadeh,P.E., Yan,B., Nguyen,M.N., Vondrusova,M., Sobol,M., Jezek,P., Hozak,P., Truksa,J., Rohlena,J., Dong,L.F., & Neuzil,J. (2017) Selective Disruption of Respiratory Supercomplexes as a New Strategy to Suppress Her2high Breast Cancer. Antioxid.Redox.Signal., 26, 84-103) (Table 6) and Seahorse instrument (Table 7). For Seahorse analysis, 20.000 or 30.000 cells were seeded in 96 well plates and incubated with compound 2 for 1 hour. Afterwards, oxygen consumption rate (OCR) and extracellular acidification rate (ECAR) were measured in three cycles of 3 minutes preceded by 3 minutes of mixing, using a Seahorse XFe96 analyzer (Agilent). Results express the mean values of the three cycles.

Table 6: The effect of compound 2 on cellular respiration in MCF7 cells as measured by the OXYGRAPH instrument. Numbers represent mean $O_2$ flow per cells ($pmol*s^{-1}*ml^{-1}$) while numbers in brackets represent standard deviations.

| Time | MCF7 |
|---|---|
| Control | 22.8 (0.5) |

(continued)

| Time | MCF7 |
|---|---|
| compound 2 - 5 μM | 15.9 (10) |
| compound 2 - 25 μM | 7.1 (0.3) |
| compound 2 - 5 μM (5h) | 9.8 (0.1) |

Table 7: The effect of compound 2 on oxygen consumption rate (OCR) and extracellular acidification rate (ECAR) in MCF7 cells as measured by the Seahorse instrument. Numbers represent mean Oxygen consumption rate (OCR; pmol*$min^{-1}$ 10.000 $cells^{-1}$) or mean extracellular acidification rate (ECAR; mpH*$min^{-1}$ 10.000 $cells^{-1}$) while numbers in brackets in both cases represent standard deviations.

| Concentration | MCF7 | |
|---|---|---|
| | OCR | ECAR |
| Control | 38.4 (3.5) | 4.6 (1.1) |
| compound 2 - 5 μM | 15.4 (3.4) | 9.69 (1.2) |
| compound 2 - 10 μM | 9.6 (0.5) | 8.2 (0.6) |

Example 23

Since compound 2 is an iron chelator, we have evaluated the effect of pretreating compound 2 with iron. at a 1:1 ratio by using the Annexin V/PI staining as in example 7. Such pretreatment significantly reduced the extent of cell death induced by this chelator, confirming that the iron-chelating properties are important for its action in the induction of cell death (Table 8).

Table 8: The effect of iron preloading on the efficacy of compound 2 to kill cancer cells (MCF7, T47D) as measured by the AnnexinV/PI staining, performed identicaly as in example 7, after 48hrs of incubation. Numbers represent mean percentage of dead cells while numbers in brackets represent standard deviations.

| Concentration | MCF7 | | T47D | |
|---|---|---|---|---|
| | compound 2 | compound 2-$Fe^{+3}$ | compound 2 | compound 2-$Fe^{+3}$ |
| 0 μM | 7.3 (1.2) | 4.9 (1.0) | 11.0 (1.5) | 6.5 (0.5) |
| 10 μM | 43.7 (6.0) | 26.9 (9.4) | 41.2 (3.1) | 23.7 (6.2) |
| 20 μM | 64.0 (9.8) | 24.4 (1.0) | 93.6 (1.3) | 36.3 (7.7) |

Example 24

The ability of compound 2 to affect the proliferation and cell death induction in real time was monitored by the Xcelligence technology that detects changes in electrical impedance related to the cell number. In brief, special 16-well e-plates containing gold electrodes weres filled with medium, blank values were recorded, and then 5.000 cells per well were seeded in the plate and let to attach for 2 hours, followed by the addition of compounds. The electrical impedance was then monitored over time in real-time every 15 minutes. In this case a line is drawn that reflects the cell number over time. The readout is then the slope of the line, where positive values reflect that the cells proliferate over time, 0 value would represent steady state where cells do not proliferate but do not die, and negative values then represent that the cells are dying over time.. We have seen a clear inhibition of proliferation with compound 2 at 1 μM, complete block of proliferation at 2μM concentration, while the cytotoxic effect is seen at 5μM (Table 9). Only mild inhibition of proliferation was seen at longer time points with DFO, yet, the block of proliferation and cytotoxic effect were missing with the DFO in similar concentrations.Our data also confirm that compound 2 does show very mild and statistically non-significant effect on cellular proliferation of non-malignant BJ cells even at 5 μM while at the same concentration malignant MCF7 cells were already dying; this supports the selectivity of the proposed compounds.

Table 9: The effect of compound 2 and DFO on cellular proliferation in malignant (MCF7) and non malignant cells (BJ) as measured with Xcelligence technology. Data represent mean values of slope of the line that measures the relative number of cells in relation to time while numbers in brackets represent standard deviations.

| Concentration | MCF7 | | | | BJ | |
|---|---|---|---|---|---|---|
| | DFO | | compound 2 | | compound 2 | |
| | 0-15 h | 0-36 h | 0-15 h | 0-36 h | 0-15 h | 0-36 h |
| 0 μM | 1 (0.25) | 1 (0.15) | 1 (0.25) | 1 (0.15) | 1 (0.24) | 1 (0.18) |
| 1 μM | 1.02 (0.12) | 1.31 (0.10) | 0.32 (0.02) | 0.35 (0.06) | 1.14 (0.16) | 1.08 (0.14) |
| 2 μM | 1.12 (0.04) | 1.53 (0.15) | 0.40 (0.08) | -0.01 (0.24) | 1.07 (0.17) | 0.90 (0.08) |
| 5 μM | 1.01 (0.18) | 0.50 (0.13) | 0.31 (0.17) | -0.47 (0.25) | 0.83 (0.13) | 0.47 (0.24) |

Example 25

We also tested the ability of compound 2 to suppress proliferation of cancer cells by an alternative approach via the real time monitoring with a cellular analyser Juli FL. In brief, MCF7 or MDA-MB-231 cells were seeded in a 6-well plate the day before to reach approximately 20% confluence on the following day. Next day, the camera of the instrument was focused on a particular field with approximately 20% confluence and cells were continuously monitored, recording the confluence and visual appearance every 30 minutes for 48 hrs. Values were then expressed either as simple confluence at the end of the experiment or as slope of the line representing the confluence during a given period of time (Table 10). It is clear from the collected data that, in agreement with Xcelligence data, we do see inhibition of proliferation at 1μM, complete block of proliferation at 2 μM and a cytotoxic effect at 5μM. The effect is less pronounced in this case as dead cells remain in the monitored field and some are still counted as living cells by the software.

Table 10: The effect of compound 2 and DFO on cellular proliferation in malignant MCF7 and MDA-MB-231 cells as measured with continuous monitoring of cell confuence via Juli FL. Data represent mean values of cellular confluence or mean values of slope of the line that measures the relative number of cells in relation to time while numbers in brackets represent standard deviations in both cases.

| Compound 2 | MCF7 | | | MDA-MB-231 | | |
|---|---|---|---|---|---|---|
| | 0-22 h | 22-48 h | Final confluence | 0-22 h | 22-48 h | Final confluence |
| 0 μM | 0.617 (0.020) | 1.300 (0.023) | 65.38 (2.9) | 0.741 (0.008) | 1.556 (0.022) | 72.87 (1.5) |
| 1 μM | 0.655 (0.022) | 0.510 (0.014) | 53.15 (4.0) | 0.786 (0.010) | 0.934 (0.014) | 65.15 (3.3) |
| 2 μM | 0.324 (0.016) | 0.104 (0.007) | 32.94 (2.5) | 0.470 (0.006) | 0.114 (0.011) | 46.58 (2.7) |
| 5 μM | 0.317 (0.030) | -0.076 (0.010) | 26.54 (1.9) | 0.454 (0.014) | -0.057 (0.013) | 25.87 (2.4) |

Example 26

In order to test the effect of compound 2 on cellular migration, we have seeded MCF7 or MDA-MB-231 cells in 12 well plates, let them reach confluence and then introduced a scratch in the monolayer with a 10 μl pipette tip. At the same time, the cultivation medium was exchanged to a 0.05% FBS containing one, which does not support proliferation, and the migration of the cells into the "clear" scratch was continuously monitored via the JULI FL system and expressed as percentage of non-invaded region relative to the initial area. Our data demonstrate that compound 2 significantly reduces the migration of cells even at 1 μM (Table 11).

Table 11: The effect of compound 2 and DFO on cellular migration in MCF7 and MDA-MB-231 cells as measured with continuos monitoring of cell confuence JULI FL. Data represent mean percentage values of non-invaded portion of the scratch while numbers in brackets represent standard deviations.The MDA-MB-231 cells started to die in the presence of compound 2 at 48 hrs, hence the values are higher than in the 24hr measurement.

| Concentration | MCF7 | | | MDA-MB-231 | | |
|---|---|---|---|---|---|---|
| | 0 h | 24 h | 48 h | 0 h | 24 h | 48 h |
| 0 μM | 100 (4.6) | 65.9 (5.2) | 42.9 (7.9) | 100 (2.9) | 16.4 (5.2) | 7.5 (7.4) |
| 1 μM | 100 (4.1) | 78.8 (5.2) | 61.8 (5.3) | 100 (5.5) | 40.7 (8.9) | 61.7 (6.7) |
| 2 μM | 100 (4.8) | 79.2 (1.1) | 66.1 (1.1) | 100 (7.4) | 42.7 (8.0) | 76.2 (13.3) |
| 5 μM | 100 (4.4) | 83.8 (0.6) | 78.8 (0.6) | 100 (6.5) | 48.4 (8.9) | 98.5 (9.1) |

Example 27

Additionally, cells seeded identically as in example 26, were treated with 1 to 5 μM concentrations of compound 2 and movie was recorded via the Juli FL system, enabling monitoring of individual movement of cells. The movement was then analyzed by the Image J program to gain the average travelled distance within a specified time. We detected a significant reduction of the total length of travelled trajectory when cells were exposed to compound 2 (Table 12)

Table 12: The effect of compound 2 and DFO on cellular migration in MCF7 cells as measured with continuos monitoring of cell confuence via JuliFL. Data represent mean values of travelled distanceof an individual cell between frames (in μm) and speed of the movement (in μm*$min^{-1}$) while numbers in brackets represent standard deviations.

| Compound 2 concentration | MCF7 | |
|---|---|---|
| | Distance | Speed |
| Control | 4.3 (1.3) | 0.19 (0.07) |
| 1 μM | 4.4 (1.4) | 0.16 (0.06) |
| 2 μM | 3.9 (1.5) | 0.13 (0.05) |
| 5 μM | 3.1 (0.7) | 0.12 (0.07) |

Example 28

We further tested the effect of the length of the attaching polycarbon linker on the anti-cancer efficacy and we found out that shortening of the carbon polylinker dramatically reduces the efficacy of compound 2. A testing compound 3 which contains 6C chain carbon linker was synthesized and tested for the efficacy in cancer killing by the crystal violet assay, exactly as described in example 18 (Table 13).

Table 13: The effect of compound 3 on cellular viability in malignant cell lines (MCF7, MDA-MB-231 and BT474) as measured with crystal violet assay. Data represent mean peracentage values of surviving cells compared to non-treated controls while numbers in brackets represent standard deviations

| Concentration | MCF7 | MDA-MB-231 | BT474 |
|---|---|---|---|
| 0 μM | 100 (5.3) | 100 (3.7) | 100 (3.4) |
| 4 μM | 100.4 (7.9) | 97.7 (8.4) | 101.7 (4.1) |
| 6 μM | 99.1 (7.5) | 95.3 (13.8) | 109.1 (7.8) |
| 8 μM | 98.2 (5.3) | 103.1 (4.3) | 104.1 (4.0) |
| 10 μM | 93.9 (9.7) | 97.4 (3.7) | 99.2 (10.3) |
| 20 μM | 84.0 (10.5) | 98.9 (2.5) | 91.5 (9.2) |
| 40 μM | 74.6 (6.1) | 91.3 (5.0) | 74.2 (7.1) |

(continued)

| Concentration | MCF7 | MDA-MB-231 | BT474 |
|---|---|---|---|
| 60 μM | 55.2 (8.5) | 86.5 (3.8) | 58.9 (8.1) |
| 80 μM | 43.2 (8.1) | 77.2 (2.5) | 49.6 (10.4) |
| 100 μM | 37.9 (6.5) | 76.0 (5.0) | 47.8 (17.1) |
| $IC_{50}$ (μM) | 70.4 (4.6) | 232.1 (26.5) | 83.5 (9.9) |

Example 29

To test the ability of the compound 2 to bring toxic compounds selectively into the cancer cells, cells were seeded at 100.000 per well of 12-well plate and the next day 10 μM compound 2 was added alone (ratio 0:1) or loaded with gallium nitrate or gallium chloride complexed with compound 2 in various ratios (ratio Ga:compound 2 1:5, 1:2, 1:1), or in the presence of 5μM gallium nitrate or chloride alone (ratio 1:0). We have then analyzed the percentage of death cells by the annexinV/PI staining as described in example 7. We have observed a significant potentiation in cell death induction when gallium was combined with compound 2 (Table 14)

Table 14

| Ratio Ga : compound 2 | MCF7 | | BJ | |
|---|---|---|---|---|
| | $GaCl_3$ | $Ga(NO_3)_2$ | $GaCl_3$ | $Ga(NO_3)_2$ |
| 1:0 | 5.9 (2.5) | 7.4 (0.8) | 2.4(0.1) | 14.45 (5.6) |
| 0:1 | 35.3 (0.5) | 35.3 (0.5) | 9.0 (3.5) | 9.0(3.5) |
| 1:5 | 57.7 (1.0) | 47.6 (5.3) | 4.6 (0.2) | 10.1 (5.8) |
| 1:2 | 55.9 (4.3) | 50.2 (0.9) | 4.6 (0.1) | 4.5 (0.2) |
| 1:1 | 63.7 (2.9) | -- | 2.8 (0.5) | -- |

Example 30

In order to define the effect of compound 2 on tumor growth and progression *in vivo,* we tested its effect on the model of syngeneic murine triple negative breast cancer cells line 4T1, Balb/c gamma mice being the immune deficient host. Mice were injected with 1 milion of cells s.c. on the right flank. After appearance of tumors (15-60 $mm^3$) mice were randomized into two separate group, one receiving corn oil (control) and one receiving compound 2 at 8 mg/kg. Tumor progression was then monitored by ultrasound imaging Vevo770 and mice received the treatment twice per week, i.p. in approximately 100 ul of corn oil. The results (Table 15) show that the triple negative breast cancer cells are markedly inhibited by the dose of 8 mg/kg which significantly reduces relative tumor growth. Each group contained at least six mice and data show mean and standard error of mean in the brackets.

Table 15: Relative tumor sizes in mice of control group and treated by Compound 2

| Time | Control | Compound 2, 8 mg/kg |
|---|---|---|
| Day 0 | 1.0 (0.0) | 1.0 (0.0) |
| Day 4 | 1.4 (0.3) | 1.3 (0.4) |
| Day 7 | 2.0 (0.7) | 1.1 (0.9) |
| Day 11 | 4.2 (1.9) | 1.4 (1.4) |
| Day 14 | 7.3 (2.8) | 1.6 (1.5)[a] |
| Day 18 | 11.2 (4.3) | 2.5 (2.1)[a] |
| a: $p < 0.05$ relative to Control, n = 5 | | |

Example 31

In order to define the effect of compound 2 on tumor growth and progression in vivo, we tested its effect on the model of human triple negative breast cancer cells line MDA-MB-231, NOD-SCID gamma mice being the immunodeficient host. Mice were injected with 1 milion of cells s.c. on the right flank. After appearance of tumors (15-60 $mm^3$) mice were randomized into three separate group, one receiving corn oil (control) and two compound 2 in a dose of 1 mg/kg or 8 mg/kg. Tumor progression was then monitored by ultrasound imaging Vevo770 and mice received the treatment twice per week, i.p. in approximately 100 ul of corn oil. The results (Table 16) show that the triple negative breast cancer cells are inhibited by the dose of 8 mg/kg which significantly reduces relative tumor growth. Each group contained at least six mice and data show mean and standard error of mean in the brackets.

Table 16: Relative tumor sizes in mice of control group and treated by Compound 2

| Time | Control | Compound 2, 8 mg/kg |
|---|---|---|
| Day 0 | 1.0 (0.0) | 1.0 (0.0) |
| Day 4 | 1.7 (0.5) | 1.3 (0.2) |
| Day 7 | 2.8 (1.6) | 1.8 (0.4) |
| Day 11 | 4.2 (1.8) | 2.6 (0.9) |
| Day 14 | 7.1 (2.0) | 3.5 (0.7)[a] |
| Day 18 | 10.0 (2.6) | 5.3 (1.3)[a] |
| Day 21 | 12.9 (2.6) | 7.9 (1.4)[a] |
| a: $p < 0.05$ relative to Control, n = 6 | | |

Example 32

The potentiation of cytotoxic effect of compound **2** in combination with several cytostatics was evaluated. The dose-effect relationship of individual drugs and mixtures of compound **2** with paclitaxel, cis Pt (cis-platin), doxorubicin and fluorouracil was determined on human mammary gland cancer cell line MDA-MB-231 and pancreatic cell line BxPC3 by means of crystal violet assay. The linearized Median-Effect Plot of dose response line provided both parameters ($IC_{50}$; trend line *m(slope)* value) of Median-Effect Equation for individual and combination treatment [ref: Theoretical basis, experimental design, and computerized simulation of synergism and antagonism in drug combination studies; Ting-Chao Chou: Pharmacol Rev. 58(3),2006, 621-81; Erratum in Pharmacol Rev. 2007;59(1),124]:

*Median-effect equation* *Linearized Median-effect plot*

$$\left(\frac{f_a}{f_u}\right) = \left(\frac{D}{IC_{50}}\right)^m \qquad \log\left({f_a}/{f_u}\right) = m\log(D) - m\log(IC_{50})$$

| **Calculated parameters of the dose-effect relationship for MDA-MB-231 cell line** *(Crystal violet assay after 48hours of cultivation)* | | | | |
|---|---|---|---|---|
| Drug and drug combination ratio | Median-Effect Plot parameters | | Combination index values | Combination index (weighted**) |
| | *m (slope)* | *$IC_{50}$ [μM] | $CI_{50}$ $CI_{75}$ $CI_{90}$ $CI_{95}$ | |
| Compound **2** | 1.2778 | 5.8934 | - | - |
| paclitaxel | 0.5451 | 0.0189 | - | - |
| cis Pt | 1.4574 | 21.7756 | - | - |
| doxorubicin | 0.4543 | 0.3187 | - | - |

(continued)

| Calculated parameters of the dose-effect relationship for MDA-MB-231 cell line *(Crystal violet assay after 48hours of cultivation)* | | | | | |
|---|---|---|---|---|---|
| Drug and drug combination ratio | Median-Effect Plot parameters | | Combination index values | | Combination index (weighted**) |
| | *m (slope)* | *$IC_{50}$ [μM] | $CI_{50}$ $CI_{90}$ | $CI_{75}$ $CI_{95}$ | |
| Compound **2**/paclitaxel (2293:1) | 1.0345 | 3.8070 | 0.733 0.980 | 0.824 (-) | 0.887 |
| Compound **2**/cis Pt (1: 1.56) | 1.5856 | 6.0669 | 0.572 0.438 | 0.500 0.401 | 0.449 |
| Compound **2**/ doxorubicin | 1.0016 | 3.1156 | 0.866 | 0.741 | 0.876 |
| (26.4:1) | | | 0.8434 | 0.971 | |
| **Calculated parameters of the dose-effect relationship for BxPC3 cell line** *(Crystal violet assay after 48hours of cultivation)* | | | | | |
| Drug and drug combination ratio | Median-Effect Plot parameters | | Combination index values | | Combination index (weighted*) |
| | *m (slope)* | **$IC_{50}$ [μM] | $CI_{50}$ $CI_{90}$ | $CI_{75}$ $CI_{95}$ | |
| Compound **2** | 1.57 | 4.50 | - | | - |
| cis Pt | 1.59 | 4.18 | - | | - |
| Fluorouracil | 0.33 | 48.32 | - | | - |
| Compound **2**/cis Pt (1: 1.44) | 1.92 | 2.39 | 0.554 0.433 | 0.490 0.398 | 0.443 |
| Compound **2**/ Fluorouracil (1:2.72) | 0.59 | 4.55 | 0.340 (-) | 0.894 (-) | 0.709 |
| *calculated by Chou-Talalay method ; **$CI= (CI_{50}+2CI_{75}+3CI_{90}+4CI_{95})/10$ | | | | | |

Potentiation of combination effect of compound **2** was expressed for concentrations $IC_{50}$, $IC_{75}$, $IC_{90}$, $IC_{95}$ by calculation of Combination index (CI) applying Combination Index Theorem for mutually exclusive drugs. Overall CI was expressed by means of weighted average of $IC_{50}$, $IC_{75}$, $IC_{90}$, $IC_{95}$ [ref: Quantitative analysis of dose-effect relationships: the combined effects of multiple drugs or enzyme inhibitors; Ting-Chao Chou, Paul Talalay: Advances in Enzyme Regulation, Volume 22, 1984, Pages 27-55]:

$$CI = \frac{D_1}{(D_x)_1} + \frac{D_2}{(D_x)_2}$$

*CI<1 (synergism); CI = 1 (additive effect); CI >1 (antagonism)*
wherein:

$$D_x = (IC_{50})[f_a/(1-f_a)]^{\frac{1}{m}}$$

$$D_1 = (D_x)_{1,2} \times [n_1/(n_1+n_2)]$$

$$D_2 = (D_x)_{1,2} \times [n_2/(n_1 + n_2)]$$

*$D_1$ - dose of first drug in mixture; $D_2$ - dose of second drug in mixture*

Data shown in the table indicate a pronounced synergy of mixtures of compound **2** at ratios corresponding to ratios of $IC_{50}$ values of individual drugs. Compound **2**/paclitaxel (2293:1), Compound **2**/cis Pt (1:1.56), Compound **2**/doxorubicin (26.4:1). In case of MDA-MB-231 cell line, concentration of paclitaxel at $IC_{75}$ (paclitaxel) and concentration of paclitaxel at $IC_{75}$ (Compound **2**/paclitaxel 2293:1) was 0.1420 μM and 0.0048 μM respectively. This allows for 97% dose reduction of toxic Paclitaxel without diminishing the $IC_{75}$ cytotoxic effect. $IC_{75}$ (MDA-MB-231) dose reduction calculated for combination of compound **2** and the remaining cytostatics is 83% for cis Pt (42.2751 μM vs 7.3877 μM), 90% for doxorubicin (3.5789 μM vs 0.3407 μM). $IC_{50}$ (BxPC3) dose reduction calculated for combination of compound **2** and examined cytostatics is 66% for cis Pt (4.184 μM *vs* 1.411 μM), 93% for fluorouracil (48.322 μM vs 3.328 μM). Combination of Compound **2** with established anticancer active ingredients (anticancer drugs) leads to significant potentiation of cytotoxic/therapeutic outcome greatly exceeding simple additive effect defined by value CI= 1.

## Claims

1. Compound of general formula I or pharmaceutically acceptable salt thereof,

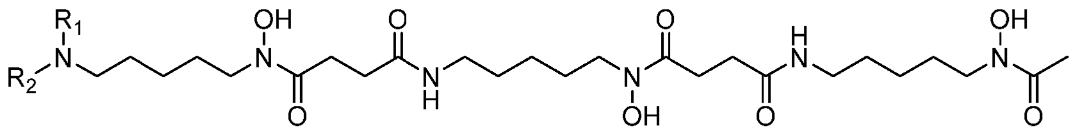
(I)

wherein R1 and R2 are independently selected from the group comprising H; C1-C6 alkyl; C6-C10 aryl; (C6-C10)aryl(C1-C6)alkyl; -C(=O)-R'; -C(=O)OR'; -C(=O)NR'R"; -C(=S)R'; -C(=S)NR'R"; wherein R' and R" are independently selected from the group comprising H, C1-C6 alkoxy, C1-C6 alkyl, C6-C10 aryl, (C1-C6)alkyl(C6-C10)aryl; whereas C1-C6 alkoxy, C1-C6 alkyl, C6-C10 aryl, (C1-C6)alkyl(C6-C10)aryl can be unsubstituted or substituted by one or more substituents selected independently from the group comprising C1-C4 alkyl, N(H or C1-C4 alkyl)$_2$, whereas alkyls are the same or different, phenyl, benzyl, OH, SH, F, Cl, Br, I, C1-C4 alkoxy, C1-C4 acyloxy, C1-C4 mercapto; and substituent of general formula II

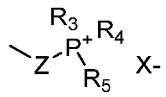
(II)

wherein Z is a linear hydrocarbyl chain selected from alkylene, alkenylene or alkynylene, containing 6 to 20 carbon atoms, preferably 6 to 16 carbon atoms, more preferably 6 to 14 carbon atoms, even more preferably 8 to 12 carbon atoms, most preferably 10 carbon atoms, or preferably 8 to 16 or 10 to 15 carbons, whereas optionally one or more carbon atoms in the hydrocarbyl chain may be replaced by one or more 5-membered or 6-membered aromatic rings or heteroaromatic rings containing the heteroatoms O, S and/or N, preferably phenylenes, triazolyls or pyridylenes, and/or one or more carbon atoms in the hydrocarbyl chain may be replaced by one or more heteroatoms or heteroatom-containing moieties selected from O, S, NH, N-OH, and whereas the hydrocarbyl chain can be unsubstituted or substituted by one or more substituents selected independently from the group comprising C1-C4 alkyl, N(H or C1-C4 alkyl)$_2$, wherein alkyls are the same or different, phenyl, benzyl, OH, =O, =N-OH, SH, =S, F, Cl, Br, I, C1-C4 alkoxy, C1-C4 acyloxy, C1-C4 mercapto,
and each of R3, R4, R5 is independently selected from the group comprising C1-C10 alkyl, C6-C12 aryl, C6-C12-aryl-C1-C2-alkyl, C5-C12 heteroaryl, C3-C8 cycloalkyl, wherein each of R1, R2, R3 can optionally (and independently from others) be substituted by one or more substituents selected independently from the group comprising C1-C4 alkyl; C1-C4 alkoxy; N(H or C1-C4 alkyl)$_2$, wherein the alkyls are the same or different; OH; =O; SH; =S; =N-OH; F; Cl; Br; I; C1-C4 mercapto,
whereas at least one of R1 and R2 is a substituent of general formula II,
and
$X^-$ is a pharmaceutically acceptable anion.

2. Compound according to claim 1, wherein R1 and R2 are independently selected from H, C1-C6 alkyl, and substituent of general formula II.

3. Compound according to any one of preceding claims, wherein R3, R4, R5 are independently selected from phenyl, benzyl, cyclohexyl, linear C1-C10 alkyl; optionally one or more of R3, R4, R5 may be further substituted by one or two substituents selected independently from the group comprising C1-C4 alkyl; C1-C4 alkoxy; OH; SH; F; Cl; Br; I; C1-C4 mercapto.

4. Compound according to any one of preceding claims, wherein

- Z is a linear hydrocarbyl chain selected from alkylene, alkenylene or alkynylene, containing 6 to 16 carbon atoms or 6 to 14 carbon atoms or 8 to 12 carbon atoms; or
- Z is a linear hydrocarbyl chain selected from alkylene, alkenylene or alkynylene, containing 6 to 16 carbon atoms or 6 to 14 carbon atoms or 8 to 12 carbon atoms, wherein one or more carbon atoms in the hydrocarbyl chain are replaced by one or more heteroatoms selected from O, S, NH; or
- Z is a linear hydrocarbyl chain selected from alkylene, alkenylene or alkynylene, containing 6 to 16 carbon atoms or 6 to 14 carbon atoms or 8 to 12 carbon atoms, wherein one or more carbon atoms in the hydrocarbyl chain are replaced by one or more 5-membered or 6-membered aromatic rings or heteroaromatic rings, preferably phenylene and/or pyridylene and/or triazole.

5. A method for preparation of the compounds of general formula I according to any one of the preceding claims, wherein a compound of general formula III

T - Z - T (III),

wherein T is halogen, mesyl, tosyl or other cleavable group and Z has the meaning as defined in claim 1, is subjected to a reaction with trisubstituted phosphine $PR_3R_4R_5$, preferably in dimethylformamide, yielding trisubstitutedphosphonium hydrocarbyl derivative of general formula IV

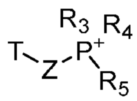
X- (IV)

which is then condensed with deferoxamine, preferably in DMF in the presence of base, preferably sodium bicarbonate, yielding the compound of general formula I.

6. Compound of formula I according to any one of claims 1 to 4 for use as a medicament, preferably in a method of treatment of proliferative diseases.

7. Compound of formula I according to any one of claims 1 to 4 for use a method of treatment of breast, prostate, GIT, hepatic, colorectal, pancreatic, mesothelioma, lung cancers, or leukaemias.

8. A pharmaceutical preparation comprising at least one compound of formula I according to any one of claims 1 to 4 and at least one metal, preferably gallium.

9. At least one compound of formula I according to any one of claims 1 to 4 and at least one metal, preferably gallium, for use in the treatment of a proliferative disease, preferably cancer, or for use as a contrast agent in diagnostic such as *in vivo* visualisation of cancer, wherein the at least one compound of formula I and the at least one metal are administered simultaneously or sequentially.

10. A pharmaceutical preparation comprising at least one compound of formula I according to any one of claims 1 to 4 and at least one further anti-cancer active ingredient, preferably selected from doxorubicin, paclitaxel, cis-platin, fluorouracil.

11. At least one compound of formula I according to any one of claims 1 to 4 and at least one further anti-cancer active ingredient, preferably selected from doxorubicin, paclitaxel, cis-platin, fluorouracil, for use in the treatment of a proliferative disease such as cancer, wherein the at least one compound of formula I and the at least one further

anti-cancer active ingredient are administered simultaneously or sequentially.

**Patentansprüche**

1. Verbindung der allgemeinen Formel I oder pharmazeutisch verträgliches Salz davon,

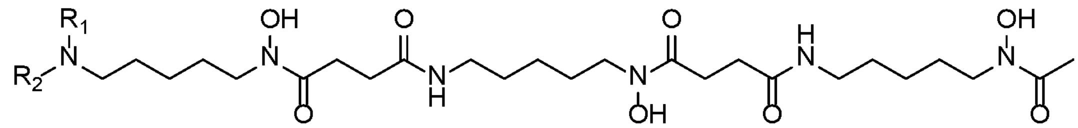 (I)

wobei R1 und R2 unabhängig ausgewählt sind aus der Gruppe umfassend H; C1-C6-Alkyl; C6-C10-Aryl; (C6-C10)Aryl(C1-C6)alkyl; -C(=O)-R'; -C(=O)OR'; -C(=O)NR'R"; -C(=S)R'; -C(=S)NR'R"; wobei R' und R" unabhängig ausgewählt sind aus der Gruppe umfassend H, C1-C6-Alkoxy, C1-C6-Alkyl, C6-C10-Aryl, (C1-C6)-Alkyl(C6-C10)aryl; wobei das C1-C6-Alkoxy, C1-C6-Alkyl, C6-C10-Aryl, (C1-C6)-Alkyl(C6-C10)aryl unsubstituiert oder durch einen oder mehrere Substituenten substituiert sein können, die unabhängig ausgewählt sind aus der Gruppe umfassend C1-C4-Alkyl, N (H oder C1-C4-Alkyl)$_2$, wobei die Alkyle gleich oder verschieden sind, Phenyl, Benzyl, OH, SH, F, Cl, Br, I, C1-C4-Alkoxy, C1-C4-Acyloxy, C1-C4-Mercapto; und Substituent der allgemeinen Formel II

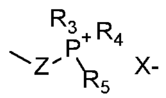 (II)

worin Z eine lineare Kohlenwasserstoffkette ist, ausgewählt aus Alkylen, Alkenylen oder Alkinylen, enthaltend 6 bis 20 Kohlenstoffatome, vorzugsweise 6 bis 16 Kohlenstoffatome, mehr bevorzugt 6 bis 14 Kohlenstoffatome, noch mehr bevorzugt 8 bis 12 Kohlenstoffatome, am meisten bevorzugt 10 Kohlenstoffatome Atome oder vorzugsweise 8 bis 16 oder 10 bis 15 Kohlenstoffatome, wobei gegebenenfalls ein oder mehrere Kohlenstoffatome in der Kohlenwasserstoffkette durch einen oder mehrere 5-gliedrige oder 6-gliedrige aromatische Ringe oder heteroaromatische Ringe mit den Heteroatomen O, S und/oder N, vorzugsweise Phenylene, Triazolyle oder Pyridylene, ersetzt sein können und/oder ein oder mehrere Kohlenstoffatome in der Kohlenwasserstoffkette durch ein oder mehrere Heteroatome oder heteroatomhaltige Einheiten ausgewählt aus O, S, NH, N-OH ersetzt sein können, und wobei die Kohlenwasserstoffkette kann unsubstituiert oder durch einen oder mehrere Substituenten substituiert sein, die unabhängig ausgewählt sind aus der Gruppe umfassend C1-C4-Alkyl, N(H oder C1-C4-Alkyl)$_2$, wobei die Alkyle gleich oder verschieden sind, Phenyl, Benzyl, OH, = O, =N-OH, SH, =S, F, Cl, Br, I, C1-C4-Alkoxy, C1-C4-Acyloxy, C1-C4-Mercapto,
und jedes von R3, R4, R5 unabhängig ausgewählt ist aus der Gruppe umfassend C1-C10-Alkyl, C6-C12-Aryl, C6-C12-Aryl-C1-C2-alkyl, C5-C12-Heteroaryl, C3-C8-Cycloalkyl, wobei jedes von R1, R2, R3 können gegebenenfalls (und unabhängig von anderen) durch einen oder mehrere Substituenten substituiert sein, die unabhängig ausgewählt sind aus der Gruppe umfassend C1-C4-Alkyl; C1-C4-Alkoxy; N(H oder C1-C4-Alkyl)$_2$, wobei die Alkyle gleich oder verschieden sind; OH; =O; SH; =S; =N-OH; F; Cl; Br; I; C1-C4-Mercapto,
wobei mindestens einer von R1 und R2 ein Substituent der allgemeinen Formel II ist,
und
$X^-$ ist ein pharmazeutisch verträgliches Anion.

2. Verbindung nach Anspruch 1, wobei R1 und R2 unabhängig ausgewählt sind aus H, C1-C6-Alkyl und Substituent der allgemeinen Formel II.

3. Verbindung nach einem der vorhergehenden Ansprüche, wobei R3, R4, R5 unabhängig ausgewählt sind aus Phenyl, Benzyl, Cyclohexyl, linearem C1-C10-Alkyl; gegebenenfalls können eines oder mehrere von R3, R4, R5 weiter substituiert sein durch einen oder zwei Substituenten, die unabhängig ausgewählt sind aus der Gruppe umfassend C1-C4-Alkyl; C1-C4-Alkoxy; OH; SH; F; Cl; Br; I; C1-C4-Mercapto.

4. Verbindung nach einem der vorhergehenden Ansprüche, wobei

- Z eine lineare Kohlenwasserstoffkette ist, ausgewählt aus Alkylen, Alkenylen oder Alkinylen, die 6 bis 16

Kohlenstoffatome oder 6 bis 14 Kohlenstoffatome oder 8 bis 12 Kohlenstoffatome enthält; oder
- Z eine lineare Kohlenwasserstoffkette ausgewählt aus Alkylen, Alkenylen oder Alkinylen ist, die 6 bis 16 Kohlenstoffatome oder 6 bis 14 Kohlenstoffatome oder 8 bis 12 Kohlenstoffatome enthält, wobei ein oder mehrere Kohlenstoffatome in der Kohlenwasserstoffkette durch eines oder mehrere Heteroatome ausgewählt aus O, S, NH ersetzt sind; oder
- Z eine lineare Kohlenwasserstoffkette ausgewählt aus Alkylen, Alkenylen oder Alkinylen ist, die 6 bis 16 Kohlenstoffatome oder 6 bis 14 Kohlenstoffatome oder 8 bis 12 Kohlenstoffatome enthält, wobei ein oder mehrere Kohlenstoffatome in der Kohlenwasserstoffkette durch eines oder mehrere 5- oder 6-gliedrige aromatische Ringe oder heteroaromatische Ringe, vorzugsweise Phenylen und/oder Pyridylen und/oder Triazol, ersetzt sind.

**5.** Verfahren zur Herstellung der Verbindungen der allgemeinen Formel I nach einem der vorhergehenden Ansprüche, wobei
eine Verbindung der allgemeinen Formel III

$$T - Z - T \qquad (III),$$

worin T ist Halogen, Mesyl, Tosyl oder eine andere spaltbare Gruppe, und Z die in Anspruch 1 definierte Bedeutung hat,
wird mit trisubstituiertem Phosphin $PR_3R_4R_5$, vorzugsweise in Dimethylformamid, umgesetzt, wodurch man trisubstituiertes Phosphoniumhydrocarbylderivat der allgemeinen Formel IV erhält

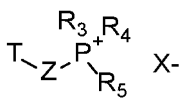
(IV)

welches dann mit Deferoxamin, vorzugsweise in DMF in Gegenwart einer Base, vorzugsweise Natriumbicarbonat, kondensiert wird, um die Verbindung der allgemeinen Formel I zu ergeben.

**6.** Verbindung der Formel I nach einem der Ansprüche 1 bis 4 zur Verwendung als Arzneimittel, vorzugsweise in einem Verfahren zur Behandlung von proliferativen Erkrankungen.

**7.** Verbindung der Formel I nach einem der Ansprüche 1 bis 4 zur Verwendung eines Verfahrens zur Behandlung von Brust-, Prostata-, GIT-, Leber-, Kolorektal-, Pankreas-, Mesotheliom-, Lungenkrebs oder Leukämien.

**8.** Pharmazeutische Zusammensetzung enthaltend mindestens eine Verbindung der Formel I nach einem der Ansprüche 1 bis 4 und mindestens ein Metall, vorzugsweise Gallium.

**9.** Mindestens eine Verbindung der Formel I nach einem der Ansprüche 1 bis 4 und mindestens ein Metall, vorzugsweise Gallium, zur Verwendung bei der Behandlung einer proliferativen Erkrankung, vorzugsweise Krebs, oder zur Verwendung als Kontrastmittel in der Diagnostik wie *in-vivo* Visualisierung von Krebs, wobei die mindestens eine Verbindung der Formel I und das mindestens eine Metall gleichzeitig oder nacheinander verabreicht werden.

**10.** Pharmazeutische Zusammensetzung enthaltend mindestens eine Verbindung der Formel I nach einem der Ansprüche 1 bis 4 und mindestens einen weiteren Anti-Krebs-Wirkstoff, vorzugsweise ausgewählt aus Doxorubicin, Paclitaxel, cis-Platin, Fluorouracil.

**11.** Mindestens eine Verbindung der Formel I nach einem der Ansprüche 1 bis 4 und mindestens ein weiterer Anti-Krebs-Wirkstoff, vorzugsweise ausgewählt aus Doxorubicin, Paclitaxel, Cis-Platin, Fluorouracil, zur Verwendung bei der Behandlung eines proliferativen Krankheit wie Krebs, wobei die mindestens eine Verbindung der Formel I und der mindestens eine weitere Anti-Krebs-Wirkstoff gleichzeitig oder nacheinander verabreicht werden.

## Revendications

**1.** Composé de formule générale I ou sel pharmaceutiquement acceptable de celui-ci,

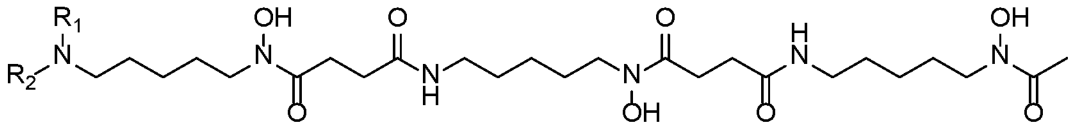

(I)

où R1 et R2 sont choisis indépendamment dans le groupe comprenant H; alkyle en C1-C6; aryle en C6-C10; (C6-C10)aryl(C1-C6)alkyle; -C(=O)-R'; -C(=O)OR'; -C(=O)NR'R"; -C(=S)R'; -C(=S)NR'R"; où R' et R" sont choisis indépendamment dans le groupe comprenant H, alcoxy en C1-C6, alkyle en C1-C6, aryle en C6-C10, (C1-C6)alkyl(C6-C10)aryle; où les substituants alcoxy en C1-C6, alkyle en C1-C6, aryle en C6-C10, (C1-C6)alkyl(C6-C10)aryle peuvent être non substitués ou substitués par un ou plusieurs substituants choisis indépendamment dans le groupe comprenant C1-C4 alkyle, N (H ou alkyle C1-C4)$_2$, où les alkyles sont identiques ou différents, phényle, benzyle, OH, SH, F, Cl, Br, I, alcoxy C1-C4, acyloxy C1-C4, mercapto C1-C4; et substituant de formule générale II

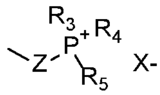

(II)

où Z est une chaîne hydrocarbyle linéaire choisie parmi alkylène, alcénylène ou alcynylène, contenant 6 à 20 atomes de carbone, de préférence 6 à 16 atomes de carbone, plus préférablement 6 à 14 atomes de carbone, encore plus préférablement 8 à 12 atomes de carbone, le plus préférablement 10 atomes de carbone, ou de préférence 8 à 16 ou 10 à 15 carbones, où éventuellement un ou plusieurs atomes de carbone dans la chaîne hydrocarbyle peuvent être remplacés par un ou plusieurs cycles aromatiques à 5 ou 6 atomes ou cycles hétéroaromatiques à 5 ou 6 atomes contenant les hétéroatomes O, S et/ou N, de préférence phénylènes, triazolyles ou pyridylènes, et/ou un ou plusieurs atomes de carbone dans la chaîne hydrocarbyle peuvent être remplacés par un ou plusieurs hétéroatomes ou fragments contenant des hétéroatomes choisis parmi O, S, NH, N-OH, et où la chaîne hydrocarbyle peut être non substituée ou substituée par un ou plusieurs substituants choisis indépendamment dans le groupe comprenant alkyle en C1-C4, N(H ou alkyle en C1-C4)$_2$, où les alkyles sont identiques ou différents, phényle, benzyle, OH, =O, =N-OH, SH, =S, F, Cl, Br, I, alcoxy en C1-C4, acyloxy en C1-C4, mercapto en C1-C4,
et chacun de R3, R4, R5 est indépendamment choisi dans le groupe comprenant un groupe alkyle en C1-C10, aryle en C6-C12, (C6-C12)aryl(C1-C2)alkyle, hétéroaryle en C5-C12, cycloalkyle en C3-C8, où chacun de R1, R2, R3 peut éventuellement (et indépendamment des autres) être substitué par un ou plusieurs substituants choisis indépendamment dans le groupe comprenant un alkyle en C1-C4 ; alcoxy en C1-C4; N(H ou alkyle en C1-C4)$_2$, où les alkyles sont identiques ou différents; OH; =O; SH ; =S; =N-OH; F; Cl; Br; I; mercapto C1-C4,
où au moins l'un de R1 et R2 est un substituant de formule générale II,
et
$X^-$ est un anion pharmaceutiquement acceptable.

2. Composé selon la revendication 1, où R1 et R2 sont indépendamment choisis parmi H, alkyle en C1-C6, et substituant de formule générale II.

3. Composé selon l'une quelconque des revendications précédentes, où R3, R4, R5 sont indépendamment choisis parmi phényle, benzyle, cyclohexyle, alkyle linéaire en C1-C10; a titre facultatif, un ou plusieurs de R3, R4, R5 peuvent être en outre substitués par un ou deux substituants choisis indépendamment dans le groupe comprenant un alkyle en C1-C4; alcoxy en C1-C4; OH; SH; F; Cl; Br; I; mercapto C1-C4.

4. Composé selon l'une quelconque des revendications précédentes, où

- Z est une chaîne hydrocarbyle linéaire choisie parmi alkylène, alcénylène ou alcynylène, contenant 6 à 16 atomes de carbone ou 6 à 14 atomes de carbone ou 8 à 12 atomes de carbone; ou
- Z est une chaîne hydrocarbyle linéaire choisie parmi alkylène, alcénylène ou alcynylène, contenant 6 à 16 atomes de carbone ou 6 à 14 atomes de carbone ou 8 à 12 atomes de carbone, où un ou plusieurs atomes de carbone dans la chaîne hydrocarbyle sont remplacés par un ou plusieurs hétéroatomes choisis parmi O, S, NH ; ou
- Z est une chaîne hydrocarbyle linéaire choisie parmi alkylène, alcénylène ou alcynylène, contenant 6 à 16 atomes de carbone ou 6 à 14 atomes de carbone ou 8 à 12 atomes de carbone, où un ou plusieurs atomes de carbone dans la chaîne hydrocarbyle sont remplacés par un ou plusieurs cycles aromatiques à 5 ou 6 atomes

ou cycles hétéroaromatiques à 5 ou 6 atomes, de préférence par phénylène et/ou pyridylène et/ou triazole.

**5.** Procédé de préparation des composés de formule générale I selon l'une quelconque des revendications précédentes, où un composé de formule générale III

T-Z-T (III),

où T est un halogène, un mésyle, un tosyle ou un autre groupe clivable et Z a la signification telle que définie dans la revendication 1, est soumis à une réaction avec de la phosphine trisubstituée $PR_3R_4R_5$, de préférence dans du diméthylformamide, donnant un dérivé hydrocarbyle de phosphonium trisubstitué de formule générale IV

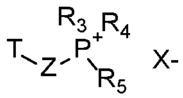

(IV)

qui est ensuite condensé avec de la déféroxamine, de préférence dans du DMF en présence de base, de préférence du bicarbonate de sodium, donnant le composé de formule générale I.

**6.** Composé de formule I selon l'une quelconque des revendications 1 à 4 pour utilisation comme un médicament, de préférence dans le traitement des maladies prolifératives.

**7.** Composé de formule I selon l'une quelconque des revendications 1 à 4 pour utilisation dans le traitement des cancers du sein, de la prostate, du tractus gastro-intestinal, hépatique, colorectal, pancréatique, du mésothéliome, du poumon, ou des leucémies.

**8.** Préparation pharmaceutique comprenant au moins un composé de formule I selon l'une quelconque des revendications 1 à 4 et au moins un métal, de préférence le gallium.

**9.** Au moins un composé de formule I selon l'une quelconque des revendications 1 à 4 et au moins un métal, de préférence le gallium, pour utilisation dans le traitement d'une maladie proliférative, de préférence le cancer, ou pour utilisation comme agent de contraste dans le diagnostic tel comme visualisation *in vivo* du cancer, où ledit au moins un composé de formule I et ledit au moins un métal sont administrés simultanément ou séquentiellement.

**10.** Préparation pharmaceutique comprenant au moins un composé de formule I selon l'une quelconque des revendications 1 à 4 et au moins un autre principe actif anticancéreux, de préférence choisi parmi la doxorubicine, le paclitaxel, le cis-platine, le fluorouracile.

**11.** Au moins un composé de formule I selon l'une quelconque des revendications 1 à 4 et au moins un autre principe actif anticancéreux, de préférence choisi parmi la doxorubicine, le paclitaxel, le cis-platine, le fluorouracile, pour utilisation dans le traitement d'une maladie proliférative telle que le cancer, où ledit au moins un composé de formule I et ledit au moins un autre ingrédient actif anticancéreux sont administrés simultanément ou séquentiellement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017079535 A

### Non-patent literature cited in the description


- **MILLER,L.D. ; COFFMAN,L.G. ; CHOU,J.W. ; BLACK,M.A. ; BERGH,J. ; D'AGOSTINO,R., JR. ; TORTI,S.V. ; TORTI,F.M.** An iron regulatory gene signature predicts outcome in breast cancer. *Cancer Research,* 2011, vol. 71, 6728-6737
- **HABASHY,H.O. ; POWE,D.G. ; STAKA,C.M. ; RAKHA,E.A. ; BALL,G. ; GREEN,A.R. ; ALESKANDARANY,M. ; PAISH,E.C. ; DOUGLAS,M.R. ; NICHOLSON,R.I.** Transferrin receptor (CD71) is a marker of poor prognosis in breast cancer and can predict response to tamoxifen. *Breast Cancer Res.Treat.,* 2009, vol. 119, 283-293
- **RYCHTARCIKOVA,Z. ; LETTLOVA,S. ; TOMKOVA,V. ; KORENKOVA,V. ; LANGEROVA,L. ; SIMONOVA,E. ; ZJABLOVSKAJA,P. ; ALBERICH-JORDA,M. ; NEUZIL,J. ; TRUKSA,J.** Tumor-initiating cells of breast and prostate origin show alterations in the expression of genes related to iron metabolism. *Oncotarget,* 2017, vol. 8, 6376-6398
- **KOH,M.Y. ; LEMOS,R., JR. ; LIU,X. ; POWIS,G.** The hypoxia-associated factor switches cells from HIF-1alpha- to HIF-2alpha-dependent signaling promoting stem cell characteristics, aggressive tumor growth and invasion. *Cancer Research,* 2011, vol. 71, 4015-4027
- **PEYSSONNAUX,C. ; ZINKERNAGEL,A.S. ; SCHUEPBACH,R.A. ; RANKIN,E. ; VAULONT,S. ; HAASE,V.H. ; NIZET,V. ; JOHNSON,R.S.** Regulation of iron homeostasis by the hypoxia-inducible transcription factors (HIFs). *Journal of Clinical Investigation,* 2007, vol. 117, 1926-1932
- **SONG,S. ; CHRISTOVA,T. ; PERUSINI,S. ; ALIZADEH,S. ; BAO,R.Y. ; MILLER,B.W. ; HURREN,R. ; JITKOVA,Y. ; GRONDA,M. ; ISAAC,M.** Wnt inhibitor screen reveals iron dependence of beta-catenin signaling in cancers. *Cancer Research,* 2011, vol. 71, 7628-7639
- **GRAZIANO,J.H.** Iron metabolism and chelation therapy in hemosiderosis. *Curr.Top.Hematol.,* 1978, vol. 1, 127-150
- **FUKUCHI,K. ; TOMOYASU,S. ; TSURUOKA,N. ; GOMI,K.** Iron deprivation-induced apoptosis in HL-60 cells. *FEBS Letters,* 1994, vol. 350, 139-142
- **SELIGMAN,P.A. ; KOVAR,J. ; GELFAND,E.W.** Lymphocyte proliferation is controlled by both iron availability and regulation of iron uptake pathways. *Pathobiology,* 1992, vol. 60, 19-26
- **SELIGMAN,P.A. ; SCHLEICHER,R.B. ; SIRIWARDANA,G. ; DOMENICO,J. ; GELFAND,E.W.** Effects of agents that inhibit cellular iron incorporation on bladder cancer cell proliferation. *Blood,* 1993, vol. 82, 1608-1617
- **WHITE,S. ; TAETLE,R. ; SELIGMAN,P.A. ; RUTHERFORD,M. ; TROWBRIDGE,I.S.** Combinations of anti-transferrin receptor monoclonal antibodies inhibit human tumor cell growth in vitro and in vivo: evidence for synergistic antiproliferative effects. *Cancer Research,* 1990, vol. 50, 6295-6301
- **ROHLENOVA,K. ; SACHAPHIBULKIJ,K. ; STURSA,J. ; BEZAWORK-GELETA,A. ; BLECHA,J. ; ENDAYA,B. ; WERNER,L. ; CERNY,J. ; ZOBALOVA,R. ; GOODWIN,J.** Selective Disruption of Respiratory Supercomplexes as a New Strategy to Suppress Her2high Breast Cancer. *Antioxid.Redox.Signal.,* 2017, vol. 26, 84-103
- **TING-CHAO CHOU.** Theoretical basis, experimental design, and computerized simulation of synergism and antagonism in drug combination studies. *Pharmacol Rev.,* 2006, vol. 58 (3), 621-81
- *Erratum in Pharmacol Rev.,* 2007, vol. 59 (1), 124
- **TING-CHAO CHOU ; PAUL TALALAY.** *Advances in Enzyme Regulation,* 1984, vol. 22, 27-55